# EUROPEAN PATENT SPECIFICATION

(11) **EP 2 841 102 B1**
(45) Date of publication and mention of the grant of the patent: **27.06.2018**
(21) Application number: 13782196.3
(22) Date of filing: 23.04.2013
(51) Int. Cl.: A61K 39/395, A01K 67/027, C07K 14/72, C12N 15/113, C07K 16/28

(54) **METHODS AND COMPOSITIONS FOR TREATING CANCER**
VERFAHREN UND ZUSAMMENSETZUNGEN ZUR BEHANDLUNG VON KREBS
MÉTHODES ET COMPOSITIONS DE TRAITEMENT DU CANCER

(30) Priority: 23.04.2012 US 201261687298 P; 05.06.2012 US 201261689378 P
(43) Date of publication of application: 04.03.2015
(73) Proprietor: Ramot at Tel Aviv University, Ltd., 61392 Tel Aviv (IL)
(72) Inventor: WERTHEIMER, Efrat, 96105 Jerusalem (IL)
(74) Representative: Schulz Junghans Patentanwälte PartGmbB
(86) International application number: PCT/IL2013/050350
(87) International publication number: WO 2013/160894

(56) References cited:
- WO-A1-2011/163430
- WO-A1-2011/163430
- WO-A1-2012/006552
- WO-A2-2005/041865
- WERTHEIMER E ET AL: "The skin specific insulin receptor knockout (SIRKO) mouse: A new outlook on diabetes complications and skin cancer", THE JOURNAL OF INVESTIGATIVE DERMATOLOGY, NATURE PUBLISHING GROUP, GB, vol. 128, no. Suppl. 1, 30 April 2008 (2008-04-30), page S141, XP008174181, ISSN: 0022-202X
- JENNY RUSS ET AL: "Impaired Insulin Signaling Is Associated with Decreased Incidence of Experimental Skin Cancer", DIABETES, AMERICAN DIABETES ASSOCIATION , vol. 56, no. Suppl. 1 30 June 2007 (2007-06-30), page A347, XP008174182, ISSN: 0012-1797 Retrieved from the Internet: URL:http://professional.diabetes.org/Abstr acts_Display.aspx?TYP=1&CID=53595 [retrieved on 2015-03-31]
- H ZHANG ET AL: "Inhibition of cancer cell proliferation and metastasis by insulin receptor downregulation", ONCOGENE, vol. 29, no. 17, 29 April 2010 (2010-04-29), pages 2517-2527, XP055180616, ISSN: 0950-9232, DOI: 10.1038/onc.2010.17
- ZHANG H ET AL: "THE THERAPEUTIC POTENTIAL OF AGENTS TARGETING THE TYPE I INSULIN-LIKE GROWTH FACTOR RECEPTOR", EXPERT OPINION ON INVESTIGATIONAL DRUGS, ASHLEY PUBLICATIONS LTD., LONDON, GB, vol. 13, no. 12, 1 December 2004 (2004-12-01), pages 1569-1577, XP008048792, ISSN: 1354-3784, DOI: 10.1517/13543784.13.12.1569
- WERTHEIMER E ET AL.: 'The skin specific insulin receptor knockout (SIRKO) mouse: A new outlook on diabetes complications and skin cancer' J INVEST DERMATOLOGY 2008 vol. 128, no. SUP.1, 30 April 2008, page S141, XP008174181
- RUSS, JENNY ET AL.: 'Impaired Insulin Signaling Is Associated with Decreased Incidence of Experimental Skin Cancer' DIABETES vol. 56, no. 1, 30 June 2007, page A347, XP008174182

## Description

### FIELD OF THE INVENTION

The present invention relates to compositions for use in a method for preventing or treating skin cancer and the recurrence of skin cancer.

### BACKGROUND

The insulin receptor (also referred to herein as IR; encoded by the insulin receptor gene; NCBI Gene ID# 3643) is a transmembranal tyrosine kinase receptor that is expressed in keratinocytes. It belongs to the IR family of proteins which includes the insulin-like growth factor receptor (IGF1R; NCBI Gene ID# 3480). Both the IR and IGF1R are ubiquitously expressed. They are composed of two alpha and two beta subunits linked by disulfide bonds. The alpha subunit is located extracellularly and contains the major hormone-binding domain. The beta subunit includes a transmembrane domain and the tyrosine kinase domain. Hormone binding to the extracellular binding domain induces receptor auto-phosphorylation that leads to activation of the IR tyrosine kinase. This initiates a signal transduction cascade, from the cell membrane to the nucleus, affecting various metabolic pathways.

Of the IR family members, transforming activity has been clearly demonstrated for the IGF1 R, which is highly homologous to the IR protein, and it is considered to be highly mitogenic. In contrast, the IR was considered a metabolic hormone involved mainly in glucose utilization and metabolism. The role of IR cellular transformation has been largely unknown and its potential usefulness as a target for anti-cancer therapy has not been explored.

Skin histology comprises an epidermis layer, which comprises keratinocytes, and a dermis layer, which comprises fibroblasts. The epidermis layer is a stratified squamous epithelium that forms the protective covering of the skin (Fuchs, 1990). Four types of layers of the epidermis are recognized on the basis of position and morphology: basal, suprabasal (spinous), granular and cornified. Proliferation takes place in the basal layer. The major products of epidermal differentiation are keratin intermediate filaments, of which keratin 5 (K5) and keratin 14 (K14) are the predominant pair expressed in the basal layer cells (Yuspa et al., 1989). In suprabasal (spinous) cells, K5 and K14 gene expression is repressed, although the proteins persist, and synthesis is initiated of a new subset of differentiation-specific keratins, keratin 1 (K1) and keratine 10 (K10), which are markers of the spinous compartment (Breitkreutz et al., 1984). K1 is encoded by the keratin 1 gene (NCBI Gene ID# 3848). In addition to keratins, maturing epidermal cells express unique proteins associated with the later stages of differentiation, such as Loricrin and Filaggrin in the uppermost granular compartment (Fuchs 1990). Filaggrin is a basic protein of the granular cell layer and comprises the major component of the keratohyaline granules (Harding and Scott, 1983).

Epithelial skin cancer is the most common cancer in the Western world. The American Cancer Society estimates that over one million new patients are diagnosed with skin epithelial cancer(non-melanoma, Squamous- and basal cell carcinomas, SCC and BCC respectively) annually, representing 50% of all invasive and *in situ* cancers. This incidence rate is rising. Epithelial skin cancer is divided into three main groups:
(1) Basal cell carcinoma (BCC) is the most common form. While it does not metastasize, it leads to destruction of tissue and bone structures if not treated.
(2) Squamous cell carcinoma (SCC) is the second most common type. It starts in the epidermis, eventually penetrating the underlying tissue and metastasizing. Furthermore, after a primary SCC, the estimated risk for any second nonmelanoma skin cancer is about 50% at five years.
(3) Actinic Keratosis is the early pre-cancerous lesions common in skin. Actinic keratosis (AK) will progress to SCC if not treated early. Most people with one actinic keratosis lesion will develop others.

The diagnosis and treatment of skin cancer at the early stages result in high rates of survival. However, there are no effective treatment options for advanced stages of skin cancer and patients do not show meaningful improvement in overall survival. As a result, distant metastases are becoming a more frequently recognized pattern of treatment failure in patients with SCC.

Furthermore, the current available treatment for skin epithelial cancer is surgical treatment that leaves scars and aesthetically disfigures the skin. As skin cancer generally involves in high rate sun exposed skin areas, such as face and hands, disfigurement is visible and is debilitating and thus is associated with high morbidity rates.

Finally, there is currently no skin-targeted treatment for the early precancerous lesions of actinic keratosis. Actinic Keratosis will progress to SCC if not treated early. Preventive treatment is preferable to treatment of cancer. The document WO2011163430 describes the preparation and characterisation of various forms of the proprietary organic compound OSI-906, whose "ability to inhibit both IGF-1 R and IR affords the special opportunity to fully target the IGF1-R/IR axis". WO2011163430 notably suggests (in claim 27) the use of OSI-906 to treat melanoma. The document by Wertheimer E et al (2008), J. Invest. Dermatol., vol.128, p.S141 describes a skin specific insulin receptor knockout (SIRKO) mouse. The document by Russ J et al (2007), Diabetes, vol.56, Suppl.1, p.A347 reports that impaired insulin signalling is associated with decreased incidence of experimental skin cancer. The document WO2012006552 describes that an anti-insulin receptor siRNA can restore sensitivity to a PI3K inhibitor in breast cancer cells , and the document by Zhang H et al (2010), Oncogene, vol.29, pp.2517-2527 describes that an anti-insulin receptor siRNA inhibits proliferation of two breast cancer cell lines. Thus, a need exists for prophylactic and post-diagnosis treatment of skin cancer and precancerous skin lesions.

### SUMMARY

The invention is as defined in the independent claim.

The present invention provides a composition as recited in the claims for use in a method as recited in the claims for preventing or treating skin cancer and precancerous lesion or preventing their recurrence.

One aspect of the present invention provides a composition as recited in the claims for use in a method as recited in the claims for treating a skin cancer or skin precancerous lesion in a subject, the method comprising the step of administering to the subject said composition that decreases insulin receptor signaling.

Another aspect of the present invention provides a composition as recited in the claims for use in a method as recited in the claims for reducing an incidence of a skin cancer or precancerous lesion in a subject, the method comprising the step of administering to the subject said composition that decreases insulin receptor signaling. Yet another aspect of the present invention provides a composition as recited in the claims for use in a method as recited in the claims for treating a skin cancer or precancerous lesion in a subject, the method comprising the step of administering to the subject said composition that decreases IR levels. As used herein "decreasing IR levels" is understood to be synonymous with "reducing IR expression." It will also be appreciated that by decreasing cellular expression of IR, IR activities (such as IR signaling) are decreased. In various embodiments, a composition for use in the present invention may decrease IR protein levels, IR activity, levels of IR messenger RNA, or levels of expression of the insulin receptor gene.

Still another aspect of the present invention provides a composition as recited in the claims for use in a method as recited in the claims for reducing an incidence of a skin cancer in a subject, via administration of said composition that decreases IR levels. In various embodiments, a composition for use in the present invention may decrease IR protein levels, IR activity, levels of IR messenger RNA, and levels of expression of the insulin receptor gene.

In certain embodiments, said composition as recited in the claims for use in a method as recited in the claims is a topical composition. Topical compositions can be a cream, or in another embodiment an ointment. They are in some embodiments particularly useful for treatment of skin cancers, for example basal cell carcinoma and squamous cell carcinoma. In various other embodiments, other routes can be utilized, for example intratumoral injection, subcutaneous injection, intradermal injection, or oral administration for example.

### BRIEF DESCRIPTION OF THE DRAWINGS

The following figures are by way of illustrative example and are not meant to be taken as limiting the claimed invention.
**Figure 1** shows IR inactivation by siRNA in human skin cells.
**Figure 2** shows the effects of IR inactivation by siRNA on proliferation of human skin cells.
**Figure 3** shows the effect of insulin on epidermal skin differentiation. **(A)** Histology of stratified epithelium in the organotypic coculture model treated with insulin or IGF1. **(B)** The effects of insulin and IGF1 on the distribution of the skin differentiation markers keratin-14 (K14), keratin-1 (K1), and Loricrin.
**Figure 4** shows the effect of IR knockout on epidermal organization in a 3-D skin organotypic coculture *in vitro.*
**Figure 5** shows the IR regulation of apoptosis in skin keratinocytes. Apoptosis of UV-irradiated IR null and control cells, as assessed by (**A,B**) caspase-3 activity and PARP cleavage analysis, and by (**C,D**) Staining for apoptotic bodies.
**Figure 6** shows the effect of insulin on cell migration, as assayed by scratch assay in murine (**A**) and human skin cells in which the insulin receptor was inactivated by siRNA (**B**).
**Figure 7** is a graph showing the effect of IR inactivation by siRNA on cellular adhesion in skin cells.
**Figure 8** shows the impaired Ras oncogene-induced transformation in IR null cells. Including changes in (**A**) Cellular proliferation rate (**B**) Growth curve (**C**) Cellular migration rate. Similar results are obtained in cells in which the IR was inactivated (**D-E**).
**Figure 9** shows impaired spindle cell formation and appearance of multi-nucleated cells in IR-null skin cells as well as in skin cells in which the IR were inactivated. Figure **9A** is a Photomicrograph of comparing WT and IR null skin cells. Figures **9B** and **9C** are charts showing quantitation of multinucleated cells. Figure **9D** is a photomicrograph showing abnormal spindle formation by immuocytochemistry.
**Figure 10** shows the effects of IR inactivation on cellular senescence. **(A)** Western blot analysis showing levels of p21 in cultured primary skin cells from control and IRKO mice. **(B)** Fluorescent microscopy of cultured primary skin cells, stained for DAPI and p21(WAF1/Cip1).
**Figure 11** shows inactivation of IR reverses ras-mediated proliferation in transformed skin cells *in vitro*. **Figure 11A** shows confirmation of IR inactivation by Western blotting. **Figure 11B** is a chart showing cell division. Cellular proliferation rate of control, Ras-transformed- and Ras-transformed IR null cells, as determined by [³H]-thymidine incorporation into cellular DNA.

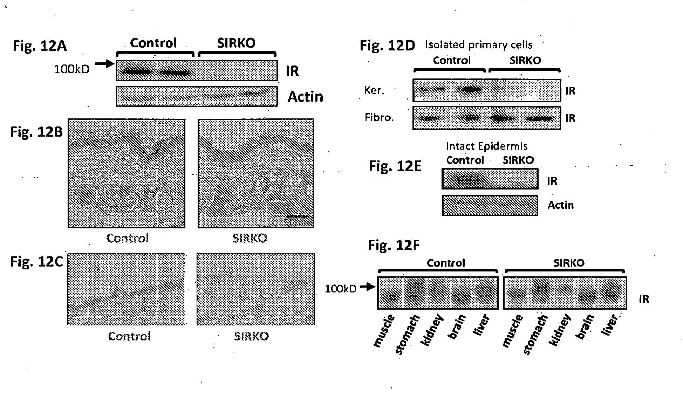

**Figure 12** demonstrates the epidermal-specific inactivation of the IR expression in a trangenic murine model *in vivo.* (**A**) IR expression in SIRKO proliferating skin cells by Western blotting. (**B**) H&E staining of skin of 2-day-old control (IRlox/lox) and SIRKO mice. (**C**) IR inactivation as demonstrated *in vivo* by immunohistochemistry in skin sections from 2-day-old control (IRlox/lox) and SIRKO mice. (**D**) Western blot analysis of protein extracts prepared from primary cultured keratinocytes and fibroblasts of 2-month-old control and SIRKO mice (two animals per genotype) using an IR-specific antiserum. (**E**) Intact epidermal layer was separated from the dermal layer of control and SIRKO mice by trypsinization, and the intact epidermises were analyzed as described in (**D**) for the expression of the IR. (**F**) Protein extracts of various tissues isolated from control and SIRKO mice were prepared and analyzed by western blot analysis.
**Figure 13** shows the inhibitory effects of IR inactivation on tumorigenesis *in vivo.* **(A)** Photos of 8-week-old control (lox+/+ cre-/-; left panel) and SIRKO (lox+/+ cre +/-; right panel) mice were shaved on the dorsal region, and a single dose of DMBA was topically applied, followed by twice-weekly application of TPA over 18 weeks. Tumor formation was quantified weekly as shown in (**B**) and (**C**).
**Figure 14** shows the effects of IR inactivation on tumor histopathology *in vivo* leading to decreased dysplasia. Representative sections of papillomas from control (**A, C, E**) and SIRKO (**B, D, F**) mice at week 18 are shown. Proliferation of cells is analyzed as well (**G**)

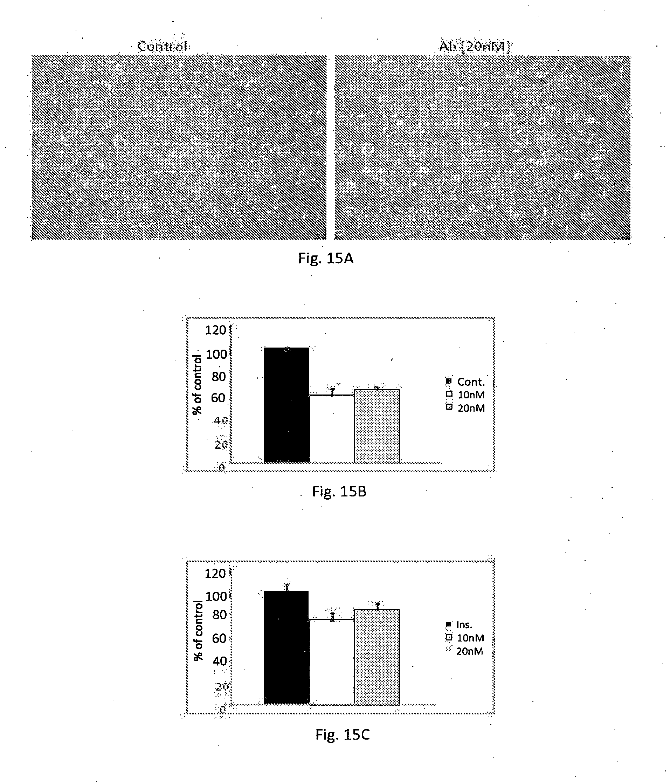

**Figure 15**. Shows the inhibitory effects of IR inhibition by an anti IR blocking antibody on proliferation of human squamous cell carcinoma (SCC) cancer cells. (**A**) a representative photograph of cells treated with 20nM anti insulin antibody for 24 hours compared to control cells. (**B**) Inhibition of IR by an anti-insulin antibody caused inhibition of SCC cancer cell proliferation. Left, center, and right bars are control, 10nM, and 20nM anti IR blocking antibody respectively. (**C**) inhibition of IR by an anti-insulin antibody caused inhibition of insulin induced SCC cancer cell proliferation. Left, center, and right bars are insulin, 10nM, and 20nM anti IR blocking antibody respectively.
**Figure 16** shows the effects of IR inactivation on epidermal turnover. (**A**) Morphology of BrdU-positive cells (dark-stained nuclei) in the back skin epidermis of control and IRKO mice were injected intraperitoneally with BrdU, after 2 h (day 0), and at 1, 3, and 5 days. (**B**) Percentage of BrdU-positive nuclei in the basal layer of the epidermis was compared to the total number of interfollicular basal cells (BrdU labeling index; vertical axis), after 2 h, and at 3 and 5 days (horizontal axis).
**Figure 17** shows the ultrastructure of cytoskeleton in the IR null epidermal skin as seen by electron microscopy analysis. Magnification is x 2,700 (A,B) or x5,000 (**C,D**). Photos show representative sections of sections processed from 10 control mice and 10 SIRKO mice.
Immunogold studies with anti-K1 antibody were performed to test whether the abnormal filaments contained keratin-1 (K1). These studies showed that K1/K10 proteins were present (Figure 17E-F).
**Figure 18** shows the impaired structure of the cytoskeleton in IR null skin cells as demonstrated by immunofluorescence microscopy. Confocal microscopy analysis showing K1 protein in primary WT (**A**) and SIRKO (**B**) murine keratinocytes is shown.
**Figure 19** shows the specific insulin regulation of K1 assembly compared to treatment with IGF1.
**Figure 20** shows the effects of lack of IR expression on K1 assembly in IR null cells. The results of a representative western blot analysis are shown (Figure **20A**). The results of Western blot analysis of the 4 repeats of each of the fractions obtained were quantified by densitometry, and the expression of K1 was normalized to actin level of each experiment individually. The combined results are presented (Figure **20B**).
**Figure 21** is a Western blot analysis showing the effects of IR inactivation on K1 assembly in murine skin cells.
**Figure 22** is a Western blot analysis showing the effect of IR inactivation by siRNA on K1 assembly in human skin cells.

### DETAILED DESCRIPTION

The invention is as defined in the claims.

### Definitions and General Techniques

Unless otherwise defined herein, scientific and technical terms used in connection with the present invention shall have the meanings that are commonly understood by those of ordinary skill of the art. Unless otherwise specified, the methods and techniques of the present invention are generally performed according to conventional methods known in the art and as described in various general and more specific references that are cited and/or discussed throughout the present specification.

The following terms, unless otherwise indicated, shall be understood to have the meanings set forth below.

As used herein "comprising" is to be interpreted as specifying the presence of the stated features, integers, steps, or components as referred to, but does not preclude the presence or addition of one or more features, integers, steps, or components, or groups thereof. Thus, for example, a pharmaceutical composition comprising a given active ingredients may contain additional ingredients. Additionally, the term "comprising" is intended to include embodiments encompassed by the terms "consisting essentially of" and "consisting of." The phrase "consisting essentially of" limits the scope of a claim to the recited materials or steps, either alone or in combination with additional materials or steps that do not materially affect the basic and novel characteristics of the claimed invention.

The term "antibody" is used herein in the broadest sense and specifically covers, for example, single monoclonal antibodies (including de-immunized, murine, chimerized or humanized antibodies), antibody compositions with polyepitopic specificity, single-chain antibodies, immunoconjugates and antibody fragments.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance witch the present invention may be made by the hybridoma (murine or human) method first described by Kohler et al., Nature, 256:495 (1975), or may be made by recombinant DNA methods (see, e.g., U.S. Pat. No. 4,816,567). The "monoclonal antibodies" may also be isolated from phage antibody libraries using the techniques described in Clackson et al., Nature, 352:624-628 (1991) and Marks et al., J. Mot Biol, 222:581-597 (1991), for example.

In accordance with the present invention, "humanized" and/or "chimeric" forms of non-human (e.g. murine) immunoglobulins refer to antibodies which contain specific chimeric immunoglobulins, immunoglobulin chains or fragments thereof (such as Fv, Fab, Fab', F(ab')₂ or other antigen-binding subsequences of antibodies) which results in the decrease of a human anti-mouse antibody (HAMA), human anti-chimeric antibody (HACA) or a human antihuman antibody (HAHA) response, compared to the original antibody, and contain the requisite portions (e.g. CDR(s), antigen binding region(s), variable domain(s) and so on) derived from said non-human immunoglobulin, necessary to reproduce the desired effect, while simultaneously retaining binding characteristics which are comparable to said non-human immunoglobulin. For the most part, humanized antibodies are human immunoglobulins (recipient antibody) in which residues from the complementarity determining regions (CDRs) of the recipient antibody are replaced by residues from the CDRs of a non-human species (donor antibody) such as mouse, rat or rabbit having the desired specificity, affinity and capacity. In some instances, Fv framework region (FR) residues of the human immunoglobulin are replaced by corresponding non-human FR residues. Furthermore, the humanized antibody may comprise residues which are found neither in the recipient antibody nor in the imported CDR or FR sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR residues are those of a human immunoglobulin consensus sequence. The humanized antibody optimally also will comprise at least a portion of an immunoglobulin constant region (Fc), typically that of a human immunoglobulin. In particular examples, antibodies for use in the methods described herein can recognize and bind specifically to the insulin receptor, such as the extracellular domain of the insulin receptor or the insulin receptor in its native 3D structure. In other examples, the antibodies can prevent insulin receptor activation by insulin or other ligands or activators.

"De-immunized" antibodies are immunoglobulins that are non-immunogenic, or less immunogenic, to a given species. De-immunization can be achieved through structural alterations to the antibody. Any de-immunization technique known to those skilled in the art can be employed. One suitable technique for de-immunizing antibodies is described, for example, in International Patent Publication No. WO 00/34317.

"Antibody fragments" comprise a portion of an intact antibody, preferably comprising the antigen-binding or variable region thereof. Examples of antibody fragments include less than full length antibodies, Fab, Fab', F(ab')₂, and Fv fragments; diabodies; linear antibodies; single-chain antibody molecules; single-chain antibodies, single domain antibody molecules, fusion proteins, recombinant proteins and multispecific antibodies formed from antibody fragment(s).

An "intact" antibody is one which comprises an antigen-binding variable region as well as a light chain constant domain (C_{L}) and heavy chain constant domains, C_{H1}, C_{H2} and C_{H3}. The constant domains may be native sequence constant domains (e.g. human native sequence constant domains) or amino acid sequence variant thereof. Preferable, the intact antibody has one or more effector functions.

The term "agent" or "active agent" is an agent that is capable of inhibiting the IR and can be, for example, an antibody or any variation thereof as described herein. The term "pharmaceutical agent or drug" as used herein refers to a chemical compound or composition capable of inducing a desired therapeutic effect when properly administered to a patient. Other chemistry terms herein are used according to conventional usage in the art.

### IR inactivation in skin cells inhibits cellular transformation

The present inventors have discovered that IR inactivation in skin cells inhibits cellular transformation. Moreover, the present inventors have discovered that the IR mediates cytoskeletal changes and they have shown that the IR is essential for cell proliferation, cellular apoptosis and initiation and maintenance of cellular transformation. The inventors have also shown that inhibiting the IR provides a method for preventing or treating skin cancer or preventing its recurrence.

Many of the studies described herein utilized models in which IR was inactivated or inhibited, to further study the role of insulin *in vitro* or *in vivo*. These IR knockout or inactivation models enabled the determination of the involvement of the IR in skin tumorigenesis both *in vitro* and *in vivo*.

The studies showed that IR-null cells exhibit a striking abnormality in the structure of K1/K10-containing intermediate filament bundles and impaired K1 intermediate filament (IF) assembly. Moreover, the studies showed that down-regulation of IR levels or disruption of IR signaling has the same effects.

### Models of In Vitro Cancer

Skin cancer development is a multistep process that can be roughly divided into initiation, promotion and progression stages. Initiation is an irreversible event in which mutations are induced in critical genes in target stem cells. Such mutations are found for example in the Ras or p53 gene. During the promotion stage, initiated cells undergo selective clonal expansion due to acquisition of proliferative advantage, and/or the ability to evade growth inhibitory or apoptotic signals. This clonal expansion of initiated cells results in the formation of benign tumors (papilloma) and enhances the probability of additional genetic mutations that might lead to malignant lesions development. The third stage of tumor progression is characterized by progression to the malignant stage resulting in skin carcinomas and metastasis.

These phases in cancer cell progression from the early pre-cancerous lesions to the final invasive stage can be also mimicked in models *in vitro*. Normal skin cells can be isolated from normal skin, where in culture, these cells proliferate and senesce following 1-3 passages *in vitro*. Furthermore, primary skin cells can be induced to differentiate similarly to the conditions *in vivo* of skin following exposure to elevated calcium concentrations and to promoters. Differentiation *in vitro* is characterized by inhibition of proliferation and increased expression of markers of differentiation including spinous K1/K10, granular Loricrin/Fillagrin and the formation of the cornified envelopes. However, when cultivating immortalized skin cell lines such as human HaCaT cells, proliferation is indefinite where cells do not senesce in culture. In addition, while HaCaT cells have the ability to differentiate in culture, differentiation is incomplete and is reversible. These cells upon grafting *in vivo*, represent skin in a hyperproliferative state with high turnover which mimics the precancerous stages of the multistage process of cancer formation.

Transformation of cells *in vitro* can be achieved by transforming them by overexpressing known oncogenes. The most recognized and studied oncogene relevant for skin cancer is Ha-ras oncogene. Thus, ras induced transformation serves as a well characterized model for studying skin cancer *in vitro*. Finally, the invasive malignant tumors can be represented by tumor cell lines which were isolated from human tumors such as the BCC and SCC lines which have basal or squamous cancer characteristics with uncontrolled proliferation along with loss of the ability to respond to physiological differentiation signals with abnormal expression of the various differentiation markers. Utilizing these cell types in culture has enabled understanding of the physiological processes which are characteristic to the different stages of cancer progression, and study of their response to curative therapies.

### Active agents

Multiple types of active agents can be used to inhibit the IR in the methods described herein of preventing and/or treating skin cancer. In certain embodiments, the active agent is an antibody, or fragment thereof retaining specific binding specificity of the antibody, which antibody or fragment thereof interacts with IR and is an IR antagonist, for decreasing IR signaling.

IR antagonist antibodies are described inter alia in Zhou et al 2008. Other examples of IR antagonist antibodies are anti-IR antibodies isolated from insulin resistance-type diabetes patients, which are available from the NIH. In other embodiments, anti-IR monoclonal antibodies are used (Zhang et al., PNAS 1991 Nov 1;88(21):9858-62). Additionally, custom-designed IR antagonist antibodies are commercially available.

In another aspect, the active agent is a small-molecule ligand that is capable of inhibiting IR activity. Small-molecule ligands of IR may be used, in certain aspects of the disclosure, for decreasing IR signaling. One example of a small-molecule IR inhibitor is human alpha2-HS glycoprotein (Mathews et al., Mol Cell Endocrinol. 2000 Jun;164(1-2):87-98). A ligand in a composition for use according to the present invention affects IR more potently than IGF1R. In more specific embodiments, the IC₅₀ (the concentration of inhibitor where the activity is reduced by half) of the ligand for IR is at least 5-fold lower than the IC₅₀ for IGF1R. Alternatively, the IC₅₀ of the ligand for IR is at least 7-fold lower than for IGF1 R. In other embodiments, the IC₅₀ of the ligand for IR is at least 10-fold lower than for IGF1 R. In still other embodiments, the IC₅₀ of the ligand for IR is at least 15-fold lower than for IGF1R. In yet other embodiments, the IC₅₀ of the ligand for IR is at least 20-fold lower than for IGF1R. In yet other embodiments, the IC₅₀ of the ligand for IR is at least 30-fold lower than for IGF1R. In yet other embodiments, the IC₅₀ of the ligand for IR is at least 50-fold lower than for IGF1R. In yet other embodiments, the IC₅₀ of the ligand for IR is at least 100-fold lower than for IGF1R.

In still other embodiments, the active agent is an insulin antagonistic mimetic compound. Such compounds are described inter alia in Schäffer et al 2003.

In certain embodiments, an antibody in a composition for use according to the present invention is a humanized antibody. In other embodiments, the antibody is a de-immunized antibody.

In other embodiments, active agents, for example antibodies, are used in combination with cell permeabilizing agents, for example as described in Weill et al. (Biotechniques. 2008 Jun;44(7):Pvii-Pxi.).

Active agents for inhibiting IR levels include in certain embodiments RNAi agents. For example, an anti-IR RNAi agent can be used to reduce expression of the insulin receptor gene and/or IR protein levels.

Non-coding RNAi molecules regulate (post-transcriptionally) genes and can lead to gene silencing. Endogenous dsRNA initiates RNAi by activating the ribonuclease protein Dicer, which binds and cleaves double-stranded RNAs (dsRNAs) to produce double-stranded fragments of 20-25 base pairs with a 2-nucleotide overhang at the 3' end. Bioinformatics and accumulating *in-vivo* studies on the genomes of multiple organisms suggest this length maximizes target-gene specificity and minimizes non-specific effects. These short double-stranded fragments are called small interfering RNAs (siRNAs). Synthetic siRNA can vary widely in their design, including the specific sequence along the mRNA, accessibility to DICER and RISC, the length of each strand, optional symmetrical, asymmetrical, blunt, and loop structures, chemical modifications of many types. These siRNAs are integrated into an active RISC complex while being separated into single "sense" and "antisense" strands. Within the RISC, the antisense strand then base-pairs to its target mRNA and induces cleavage of the mRNA, thereby preventing it from being used as a translation template.

The design of appropriate RNAi agents targeting cellular proteins is well within the ability of those skilled in the art (*see* for examples, Jansen et al, 2002; Wang et al, 2001; United States Patent No. 7,078,196), who will appreciate, in light of the present disclosure, that such agents can be used for the methods described herein. Use of RNAi agents is also described in U.S. Patent Publication Nos, US 2012/0065250; US 2012/0058188; US 2012/0065242; and US 20120029053.

Certain preferred embodiments of RNAi nucleotides are short (or small) interfering RNA (siRNA), short hairpin RNA (shRNA), and microRNA. Other preferred embodiments are molecules that are processed intracellularly to yield siRNA. Such molecules include DsiRNA, which are cleaved by the RNase III class endoribonuclease dicer into 21-23 base duplexes having a 2-base 3'-overhang; UsiRNAs, which are duplex siRNAs that are modified with non-nucleotide acyclic monomers, termed unlocked nucleobase analogs (UNA), in which the bond between two adjacent carbon atoms of ribose is removed-these may be designed to enter the RNAi (RNA inhibitory) pathway via Dicer enzyme or directly into RISC; self-delivering RNA (sdRNA) such as rxRNA™ of RXi Therapeutics, which has a single-stranded phosphorothioate region, a short duplex region, and contains a variety of nuclease-stabilizing and lipophilic chemical modifications; aptamers, triple-helix antisense nucleotides, DNAzymes; and agents inhibiting the pre-mRNA maturation step of polyA tail addition such as the U1 adaptor (Integrated DNA Technologies (IDT) Inc). The U1 adaptor consists of two parts, a target-gene binding domain and a U'1 domain that attracts and inhibits the cellular splicing apparatus. By combining both capabilities in the same molecule, the U1 adaptor can inhibit the pre-mRNA maturation step of polyA tail addition.

In certain embodiments, a composition for use according the present invention comprises an active agent that is a peptide IR antagonist, which decreases IR activation or signaling. In still other non-limiting examples, the peptide is capable of blockading the interaction between insulin and IR. In further examples, the peptides are based on phosphopeptides derived from the insulin receptor, for example those described in Herrera et al (PNAS. 1985 Dec;82(23):7899-903). Yet other non-limiting examples of peptides for use in the present invention include those such as from Aviva Systems Biology Corp, San Diego, CA. Still other examples are antagonistic mutant insulin proteins and analogues thereof, for example those described in Nanjo et al (J Clin Invest. 1986 Feb;77(2):514-9), which in some cases act as competitive inhibitors of wild-type insulin. Additional examples of peptides for inhibition of IR signaling can be found in Knudsen et al. PLOS One, 7(12), Dec, 2012.

For access to intracellular sites, a peptide in a composition for use according to the present invention is capable, in some embodiments, of entering the target cell under physiological conditions. Examples of peptides capable of entering cells are peptides that are internalized via active transport and peptide that are cell-membrane permeable via diffusion, each of which is intended to be encompassed by various embodiments of the present invention.

### Target diseases

As provided herein, IR *per se*, as opposed to the IGF1 R signaling pathway, plays a role in cellular transformation. It will be appreciated by those skilled in the art in light of the present disclosure, that compositions for use in a method according to the present invention are for use in treating, preventing, reducing the incidence of a variety of types of skin cancer and preventing their recurrence.

As used herein, the terms "cancer" refers to a physiological condition in mammals that is typically characterized by unregulated cell growth, typically referred to as a tumor, neoplasm, and/or malignancy. The cancer may be multi-drug resistant (MDR) or drug-sensitive. The unregulated growth can be a feature of benign or malignant lesions. Examples of skin cancer include but are not limited to, papilomas, carcinoma, sarcoma, and melanoma. More particular examples of skin cancer include basal cell carcinoma and squamous cell carcinoma.

Other embodiments of the present invention provide a composition as recited in the claims for use in a method as recited in the claims for reversing transformation of skin cancer and pre-cancerous growths and hyperplastic skin lesions.

In more specific embodiments, the target skin cancer is a skin cancer whose level is increased in T2D patients.

In even more specific embodiments; the target cancer is basal cell carcinoma. Alternatively the target cancer is squamous cell carcinoma.

In certain embodiments, reducing an incidence of a skin cancer as used herein refers to treatment of the skin of a subject to render the skin resistant to future tumor formation or to prevent future tumor formation. In certain embodiments, the skin is made resistant to tumor formation in general. In other embodiments, the skin is rendered resistant to one or more environmental inducers of cancer. In more particular embodiments, the inducer is ultraviolet (UV) radiation. Alternatively, the inducer is gamma radiation. In other embodiments, the inducer is a carcinogen, e.g. arsenic. In other embodiments, the inducer is a history of sunburns. In other embodiments, the inducer is excessive sun exposure. In other embodiments, the inducer is residence in a sunny locale. In other embodiments, the inducer is residence in a high-altitude location.

In Still other embodiments, a composition for use according to the present invention is indicated for skin tumor prevention in a subject with a disease that renders a patient susceptible to skin cancer. In more specific embodiments, the subject has an immunosuppressive disease. In more specific embodiments, the disease may be HIV/AIDS. In other embodiments the disease may be leukemia. In still other embodiments, a composition for use according to the present invention is indicated for skin tumor prevention in a subject receiving immunosuppressant drugs, e.g. following an organ transplant.

In other embodiments, a composition for use according to the present invention is indicated for skin tumor prevention in a subject at increased risk for skin cancer. In more specific embodiments, the subject may have a family history of skin cancer. In other embodiments, a composition for use according to the present invention is indicated for skin tumor prevention in a subject having fair skin. In other embodiments, a composition for use according to the present invention is indicated for skin tumor prevention in a subject with actinic keratoses. In other embodiments, a composition for use according to the present invention is indicated for skin tumor prevention in a subject with many moles. In other embodiments, a composition for use according to of the present invention is indicated for skin tumor prevention in a subject with dysplastic nevi. In other embodiments, a composition for use according to the present invention is indicated for skin tumor prevention in a subject over the age of, for example, 55, 65, or over 75. In other embodiments, a composition for use according to the present invention is indicated for skin tumor prevention in a subject over the age of 65. In some embodiments, a cream or ointment comprising an agent as recited in the claims is for use in a methods as recited in the claims by administration to a skin site at risk of developing cancer.

In other embodiments, a pharmaceutical composition comprising an agent as recited in the claims is for use in a method as recited in the claims by administration to a tumor bed of an excised skin cancer, for example a squamous cell carcinoma or, in other embodiments, a basal cell carcinoma.

In other embodiments, compositions as recited in the claims for use according to the present invention are for treating, preventing, or reducing the incidence of actinic keratosis.

In still other embodiments the described compositions for use as recited in the claims are for use in methods of treating non-cancerous abnormal skin cell proliferation, for example in warts.

Additionally, as provided herein, skin turnover is pathologically accelerated by inactivation of IR in keratinocytes.

Furthermore, as provided herein, cell proliferation, as assayed by scratch assay, is impaired by inactivation of IR in keratinocytes.

In particular embodiments, the compositions for use as recited in the claims are for use in methods of treatment or prevention of pre-cancerous lesions. Typically, damaged skin cells are, either repaired or replaced by normal body processes. If not, the normal cycle of skin cells is disrupted and cells keep dividing and growing. Such lesions, also referred to as precancerous lesions therefore present a risk for cancer development. These precancerous cells look different from normal skin cells and lead to appearance of skin outgrowths, or appearance of lesions that are scaly or show changes in skin structure and color. Some examples include Actinic keratosis, Actinic chelitis, and Leukoplakia.

Several risk factors are associated with induction of skin damage which predisposes the skin to precancerous lesions, including sun exposure, exposure to environmental hazards, radiation treatment, aging and even heredity.

It is demonstrated herein that in addition to known proliferative factors, insulin has direct effects on the proliferation, differentiation, apoptosis and turnover of skin or on the interaction of skin cells with its basement membrane, both of which processes are found to be abnormal and characteristic for changes which occur in cancer formation. As described in this invention, it is for this reason that diabetes patients are prone to abnormal growth of hyperplastic skin lesions which are more common and some even distinct to the diabetes state. These lesions include skin tags, Necrobiosis lipoidica diabeticorum (NLD), Acquired Perforating Dermatosis, Scleredema Diabeticorum, Acanthosis Nigricans (AN), Diabetic Dermopathy and Bullosis Diabeticorum . These lesions which involve an outgrowth of skin cells and abnormal regulation of skin proliferation and differentiation together with the immunopathology reaction typical to diabetes are associated with insulin deregulated states as is characteristic to the pre diabetes and diabetic state. These and similar skin lesions can all be treated or even prevented by the method described herein.

### Target tissues

Typically, the target of compositions as recited in the claims for use according to the present invention will be one or more cells of the subject in need of treatment for one or more of the indicated disease conditions. In certain embodiments, the target cells are keratinocytes skin cells. In other embodiments, the target cells are one or more other cell types that express IR.

In other embodiments, the target tissue is skin. In other embodiments, the target tissue may be any other type of body tissue.

### Therapeutic Methods of Use

According to some embodiments, the invention provides a composition as recited in the claims for use in a method as recited in the claims and inhibiting IR activity upon administration of the active agent to a patient to prevent or treat a target disease or prevent its recurrence. Any of the types of agents as recited in the claims may be used therapeutically.

According to some embodiments, the agent is an anti-IR antibody, which can be a human, chimeric or humanized antibody. In other embodiments, the agent can be an siRNA molecule. In some embodiments, the anti-IR antibody is human and the patient is a human patient. Alternatively, the patient may be a mammal that expresses an IR that the anti-IR antibody cross-reacts with. The antibody may be administered to a non-human mammal expressing an IR with which the antibody cross-reacts (i.e. a primate, or a cynomologous or rhesus monkey) for veterinary purposes or as an animal model of human disease. Such animal models may be useful for evaluating the therapeutic efficacy of antibodies in a composition for use in a method of this invention.

The agent can be administered once or can be administered multiple times. The agent can be administered from three times daily to once every six months. The administering may be on a schedule such as three times daily, twice daily, once daily, once every two days, once every three days, once weekly, once every two weeks, once every month, once every two months, once every three months and once every six months. The agent can be administered via an oral, mucosal, buccal, intranasal, inhalable, intravenous, subcutaneous, intramuscular, parenteral, intratumor, or topical route. The agent can be administered at a site distant from the site of the tumor or precancerous site. The agent can also be administered continuously via a minipump. The agent can be administered once, at least twice or for at least the period of time until the condition is treated, palliated or cured. The agent can generally be administered for as long as required or desired. The effective amount of the agent can generally be administered as part of a pharmaceutical composition as described below. The dosage of agent can generally be in the range of 0.001-1mg/kg, or in the range of 0.01-1 mg/kg or in the range of 0.1-100 mg/kg, or in the range of 0.5-50 mg/kg, or in the range of 1-20 mg/kg, or in the range of 1-10 mg/kg. The serum concentration of the agent may be measured by any method known in the art. The agent can be administered prophylactically in order to prevent a cancer or tumor from occurring. This may be especially useful in patients that have a higher risk of developing one or more of the target diseases described herein.

The amount of each therapeutic (active) agent that will be effective will depend on the nature of the disease or condition to be treated, as well as the stage of the disease or condition. Effective amounts can be determined by standard clinical techniques. The precise dose to be employed in the formulation will also depend on the route of administration, and should be decided according to the judgment of the health care practitioner and each patient's circumstances. Exemplary dosages of the individual compounds are described herein, but myriad other dosage regimens are encompassed by this disclosure.

The specific dose level and frequency of dosage for any particular subject may be varied and will depend upon a variety of factors, including the activity of the specific compound, the metabolic stability and length of action of that compound, the age, body weight, general health, sex, diet, mode and time of administration, rate of excretion, drug combination, and severity of the condition of the host undergoing therapy.

In another aspect, the agent can be co-administered with other therapeutic agents, such as antineoplastic drugs or molecules, to a patient who has a target disease as described herein. In one aspect, the invention relates to a composition as recited in the claims for use in a method as recited in the claims in a mammal comprising administering to said mammal a therapeutically effective amount of an agent as recited in the claims in combination with an anti-tumor agent selected from the group consisting of, but not limited to, mitotic inhibitors, alkylating agents, antimetabolites, intercalating agents, growth factor inhibitors, cell cycle inhibitors, enzymes, topoisomerase inhibitors, biological response modifiers, anti-hormones, kinase inhibitors, matrix metalloprotease inhibitors, genetic therapeutics and anti-androgens. In some embodiments, the agent can be administered with an antineoplastic agent, such as adriamycin or taxol. In another preferred embodiment, the agent or combination therapy can be administered along with radiotherapy, chemotherapy, photodynamic therapy, surgery or other immunotherapy. In yet other embodiments, the agent can be an anti-IR antibody and be administered with another antibody. For example, the anti-IR antibody can be administered with an antibody or other agent that is known to inhibit tumor or cancer cell proliferation, e.g., an antibody or agent that inhibits erbB2 receptor, EGF-R, CD20 or VEGF.

Co-administration of the agent with an additional therapeutic agent (combination therapy) encompasses administering a pharmaceutical composition comprising the agent and the additional therapeutic agent and administering two or more separate pharmaceutical compositions, one comprising the agent and the other(s) comprising the additional therapeutic agent(s). Further, although co-administration or combination therapy generally means that the agent and additional therapeutic agents are administered at the same time as one another, it also encompasses instances in which the agent and additional therapeutic agents are administered at different times. For instance, the agent can be administered once every three days, while the additional therapeutic agent is administered once daily. Alternatively, the agent can be administered prior to or subsequent to treatment of the disorder with the additional therapeutic agent. Similarly, administration of the agent can be administered prior to or subsequent to other therapy, such as radiotherapy, chemotherapy, photodynamic therapy, surgery or other immunotherapy.

The agent and one or more additional therapeutic agents (the combination therapy) can be administered once, twice or at least the period of time until the condition is treated, palliated or cured. The combination therapy can be administered multiple times. The combination therapy can be administered from three times daily to once every six months. The administering can be on a schedule such as three times daily, twice daily, once daily, once every two days, once every three days, once weekly, once every two weeks, once every month, once every two months, once every three months and once every six months, or can be administered continuously via a minipump. The combination therapy can be administered via an oral, mucosal, buccal, intranasal, inhalable, intravenous, subcutaneous, intradermal, intramuscular, parenteral, intratumor or topical route. The combination therapy can be administered at a site distant from the site of the tumor. The combination therapy generally can be administered for as long as the treatment is required or desired.

### Pharmaceutical compositions

Another aspect of the present invention provides a composition as recited in the claims for use in a method for treating or preventing skin cancer, a pre-cancerous or hyperplastic skin lesion, basal cell carcinoma, squamous cell carcinoma, or actinic keratosis.

Another aspect of the present invention provides a composition as recited in the claims for use in a method as recited in the claims and reducing an incidence of a skin cancer.

Still another aspect of the present disclosure provides a method of reducing an incidence of a skin cancer in a subject, via administration of a composition that decreases IR levels or activity.

Other aspects of the present disclosure provide use of a substance that decreases IR signaling in the manufacture of a medicament for treating a skin cancer.

Other aspects of the present disclosure provide use of a substance that decreases IR signaling in the manufacture of a medicament for reducing an incidence of a skin cancer.

Yet other aspects of the present disclosure provide use of a substance that decreases IR levels in the manufacture of a medicament for treating a skin cancer.

Still another aspect of the present disclosure provides a method of reducing an incidence of a skin cancer in a subject, via administration of a substance that decreases IR levels or activity.

In certain embodiments, a composition for use according to the present invention is a topical composition.

Topical compositions can be particularly useful for treatment of skin cancers, for example basal cell carcinoma and squamous cell carcinoma.

In other embodiments, a pharmaceutical composition for use according to the present invention comprises an active compound as recited in the claims and a pharmaceutically acceptable carrier and optionally other therapeutic ingredients. In various embodiments, any suitable route of administration may be employed for providing a mammal, especially a human with an effective dosage of a composition for use according to the present invention. For example, oral, intradermal, peritoneal rectal, topical parenteral, ocular, pulmonary, nasal, and the like may be employed. Dosage forms include tablets, troches, dispersions, suspensions, solutions, capsules, creams, ointments, gels, aerosols, and the like. In other embodiments, compositions for use according to the present invention include compositions suitable for oral, intradermal, peritoneal, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In yet other embodiments, for administration by inhalation, the composition for use according to the present invention may be conveniently delivered in the form of an aerosol spray presentation from pressurized packs or nebulizers. The compounds may also be delivered as powders which may be formulated and the powder composition may be inhaled with the aid of an insufflation powder inhaler device. The preferred delivery system for inhalation is a metered dose inhalation (MDI) aerosol, which may be formulated as a suspension or solution of a compound as recited in the claims in suitable propellants, such as fluorocarbons or hydrocarbons.

In still other embodiments, suitable topical formulations include transdermal devices, aerosols, creams, ointments, lotions, creams, gels, dusting powders, and the like. The active agent, is admixed under sterile conditions with a pharmaceutically acceptable carrier and any needed preservatives or buffers as may be required. Prophylactic formulations may be present or applied to the site of cancerous or pre-cancerous skin lesions. The ointments, pastes, creams, and gels may contain, in addition to an active agent as recited in the claims, excipients such as animal and vegetable fats, oils, waxes, paraffins, starch, tragacanth, cellulose derivatives, polyethylene glycols, silicones, bentonites, silicic acid, talc, zinc oxide, or mixtures thereof. Powders and sprays can contain, in addition to the agents as recited in the claims, excipients such as lactose, talc, silicic acid, aluminum hydroxide, calcium silicates, polyamide powder, or mixtures of these substances. Sprays can additionally contain customary propellants such as chlorofluorohydrocarbons. Transdermal patches have the added advantage of providing controlled delivery of the active ingredients to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

In order that this invention may be better understood, the following examples are set forth. These examples are for purposes of illustration only and are not to be construed as limiting the scope of the invention in any manner.

### EXAMPLES

### EXAMPLE 1: Insulin Receptor (IR) inactivation reduces proliferation of human skin cells

### Methods

### IR knockout mouse

Homozygous IR null nice and wild-type littermates were obtained by crossing heterozygous IR null mice, as described (Accili et al. Nat Genet 1996, 12:106-109). Mice were genotyped using PCR as described there. Animal experimentation was approved by an appropriate institutional committee.

### HaCaT cell culture

Human HaCaT epidermal keratinocytes (Boukamp et al 1988) were cultured in media containing 0.05mM Ca2⁺.

### Transfection with IR shRNA constructs

HaCaT cells were transfected by a mixture of IR shRNA constructs (GIPZ Lentiviral shRNAmir Library from Thermo Scientific Open Biosystems). The cultures were either studied after a transient transaction or stable clones (IRKD) were selected and isolated, following the manufacturer's instructions. Control cells were infected by an empty vector (control).

### Results

Insulin receptor signaling in skin can be studied inter alia using human HaCaT epidermal keratinocytes and murine skin primary epidermal keratinocytes. In these model systems, primary keratinocytes cultured in low Ca²⁺ concentration (0.05mM) medium retain a proliferative basal cell phenotype. Elevation of the Ca²⁺ concentration induces a rapid sequence of events, leading to the cessation of cell proliferation and induction of differentiation (Breitkreutz et al, 1993; Fuchs 1990; Yuspa et al, 1989). Induction of differentiation is associated, similarly to the differentiation of skin *in vivo*, with induced expression of the differentiation markers, as well as cornified envelope formation and cell death (Fuchs 1990; Yuspa et al, 1989).

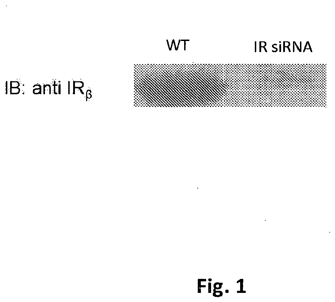

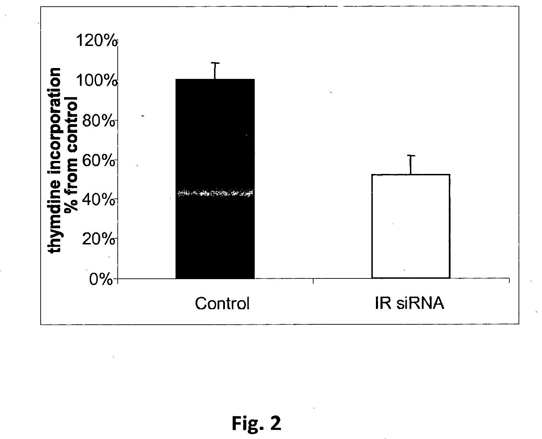

In the first experiment, HaCaT cells were transfected by a mixture of IR shRNA constructs, and stable clones (IR siRNA) were selected and isolated and compared to control clones (control). It was shown that IR inactivation could be achieved in human skin cells by transfecting the cells with IR-shRNA plasmids (Figure 1). Knockdown of this gene resulted in a decrease in proliferation as measured by thymidine incorporation (Figure 2). In this experiment thymidine incorporation of human control and siRNA inactivated (IR siRNA) HaCaT cells was performed. The proliferation rate of the control cells was set as 100%. A representative experiment out of 4 is shown.

### EXAMPLE 2: The IR is necessary for differentiation of human keratinocytes

### Methods

### 3D skin organotypic coculture

Primary keratinocytes and fibroblasts were isolated from WT mice, and organotypic cocultures were plated. Immediately afterwards, 10⁻⁶M insulin or 10⁻⁷M IGF1 was added to the growth medium and maintained throughout the experiment. Coculture inserts were further processed; H&E staining of a tissue section is shown.

### Results

The effects of insulin and IGF1 on differentiation of human keratinocytes were tested in a 3-dimensional organotypic skin co-culture. In this model system, primary murine or human keratinocytes were plated on top of a fibroblast-containing collagen gel dermal equivalent. Within a few days under proper growth conditions, the 3D-organotypic coculture became organized, mimicking the histological structure of the skin tissue *in vivo*, including the skin epidermal layers as well as keratin formation. By mimicking the natural tissue architecture, such conditions enable keratinocytes to recapitulate their tissue-specific differentiation program *in vitro*.

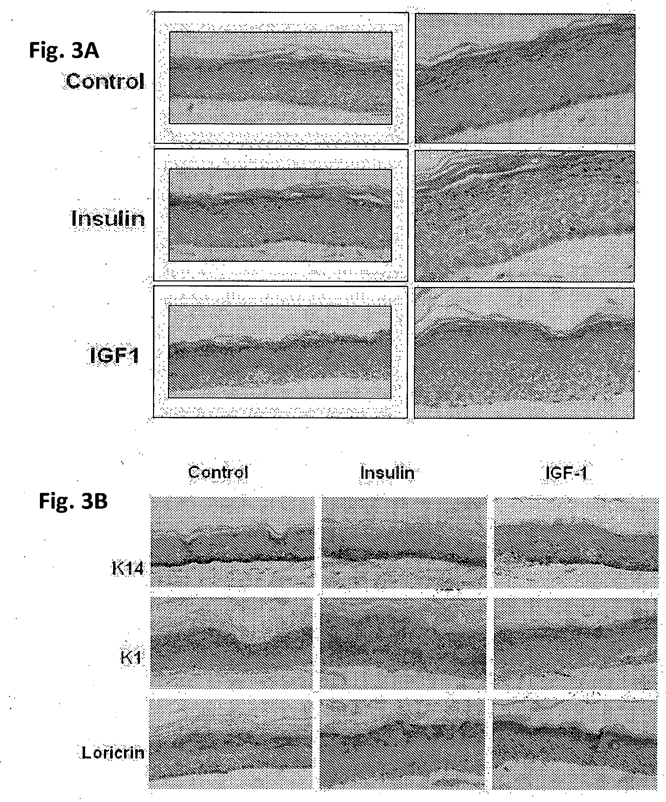

Insulin treatment in this model system supported epidermal differentiation both by histological analysis (Fig 3A), as well as by following the expression of epidermal differentiation markers by immunohistochemical analysis. Insulin resulted in a more rapid disappearance of the expression of K14, which is a marker of proliferating keratinocytes, while the expression of the differentiation markers Keratin-1 and Loricrin was enhanced (Fig 3B). In contrast, pretreatment with IGF1 resulted in delayed expression of the differentiation markers, while the expression of keratin 14 was maintained abnormally in apical layers of the organotypic coculture.

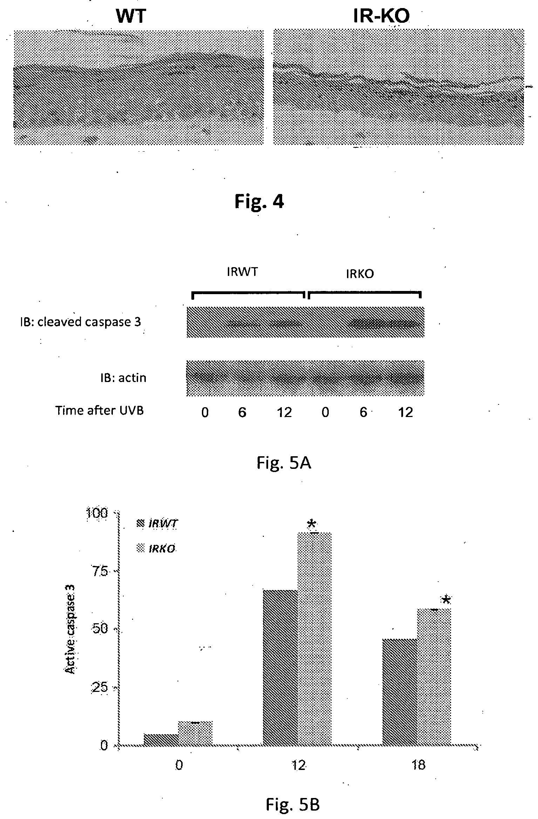

In a subsequent experiment, a 3-D skin organotypic coculture established from IR-KO cells is depicted. Cocultures were established as described above, from primary cells isolated from IRKO mice (produced as described in Example 1) and WT littermates. These cultures exhibited thinning of skin with reduced proliferation and differentiation of the *in vitro*-formed skin (Figure 4).

### EXAMPLE 3: IR inactivation increases apoptosis of skin cells

### Methods

### Experimental apoptosis induction protocol

Primary cells from the IR knockout mouse and control cells were grown in culture. On the day of the experiment, the cells were UVB-irradiated using the CROSSLINKER CL-508 (UVITEC). 12h after radiation, apoptosis was followed by annexin activation (APOPTOSIS KIT Cat#4700, MBL, Japan); caspase 3 activity was measured by Western blotting using Monoclonal antiHuman/mouse cleaved caspase - 3 Asp 175 Cat#MAB835. R&D system, Minneapolis, MN, USA; and PARP cleavage analysis was measured by Western blot using PARP Antibody, 9542, Cell Signaling Technology. A representative experiment out of 3 is shown in Figure 5A. Quantitation of all experiments is shown in figure 5B.

### Quantification of apoptotic bodies

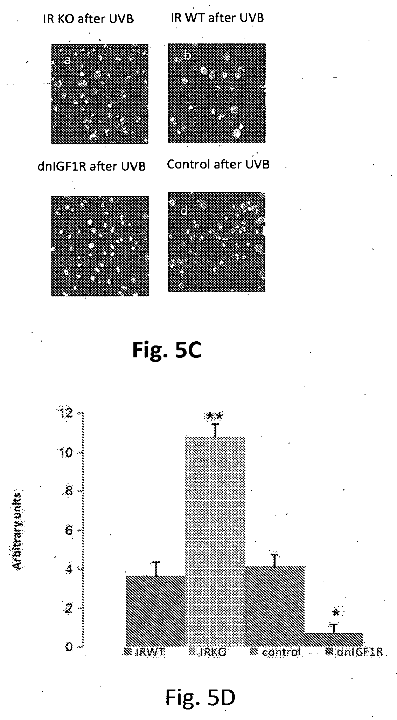

Cell cultures of the following were studied: Primary cells were isolated from IR KO; IR WT control; cells over-expressing a dominant negative construct of IGF1R (DN IGF1R); or cells infected with an adenoviral null construct (control). Cells were grown on coverslips to confluency. On the day of the experiment they were UVB irradiated and further incubated for the time indicated above. Cells were fixed with paraformaldehyde and stained with DAPI (4,6'-diamidino-2-phenylindole, dihydrochloride, Cat#71-03-00, KPL, Gaithersburg, MD, USA). Photographs were taken from arbitrarily chosen fields with a Leica TCS SC5 microscope (x63) (shown in Figure 5C), and the number of apoptotic bodies was evaluated. The quantization is shown in figure 5D.

### Results

To test the effect of IR inactivation on keratinocyte apoptosis, primary cells from the IR knockout mouse and control cells were grown in culture and UVB irradiated. Irradiation resulted in increased apoptosis in IR-null cells compared to WT control cells, as shown by caspase-3 activity and.PARP cleavage analysis (Figures 5A,B) and by quantification of the number of apoptotic bodies (Figures 5C,D).

### EXAMPLE 4: Insulin treatment enhances skin cell migration

### Methods

Insulin and IGF1 treatment: Primary murine keratinocytes were grown for 6 days in culture. On the day of the experiment rinsulin (10⁻⁶M) or IGF-1 (10⁻⁷M) were added, after which a scratch was made by a micropipette tip (day 0). In some of the plates, proliferation was inhibited by adding 25 microgram/ml mitomycin C, 4 hours prior to culture wounding. In another set of experiments (E-F) insulin receptor was inactivated by siRNA in human HaCaT skin cells. The effects of insulin and IGF1 were measured as described above. In all experiments digital photos were taken at day 0 and 24 hours later (day 1), from which 20 randomly chosen photos were taken from each treatment group and further analyzed to determine the average remaining scratch width.

### Results

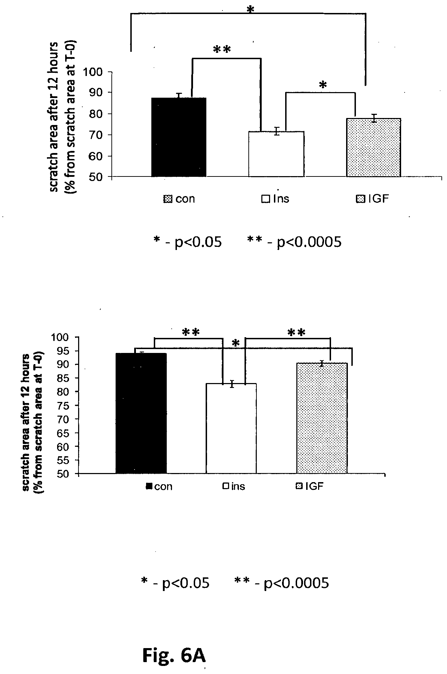

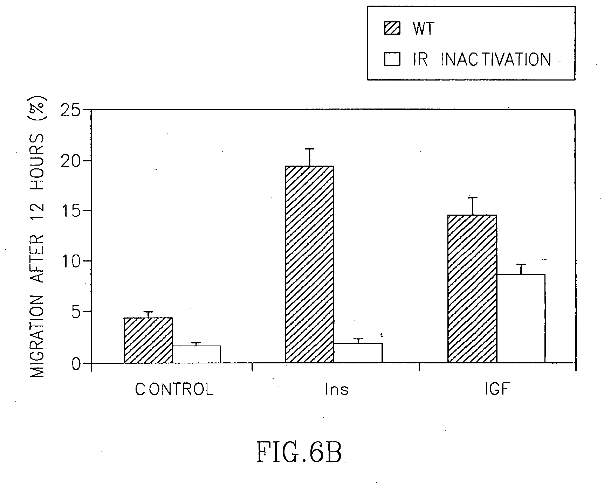

The effect of insulin on skin cell migration was assessed using an *in vitro* scratch closure assay. Keratinocytes were grown to confluency, were left untreated or were treated with insulin or IGF-1, in the absence (6A; top panel) or presence (6A; bottom panel) of mitomycin C. The cell layer was scratched by a tip, and the degree of closure was subsequently measured. The amount of migration of control cells in each group (with or without mitomycin C) was assigned the value 100%. The average of 4 separate experiments is depicted. Insulin treatment facilitated scratch closure more than IGF (Figure 6A, top panel). This effect was not eliminated by the cell proliferation inhibitor mitomycin C, demonstrating that the effects of insulin are on cellular migration and not only on cell proliferation (Figure 6A, bottom panel). Similar results were obtained in human HaCaT skin cells in which the insulin receptor was inactivated by siRNA. Furthermore, the effect of insulin, more so than IGF-1, was eliminated by IR inactivation (Figure 6B). These data show that these effects of insulin are mediated distinctly via IR and is distinct from IGF1 induced effects..

### EXAMPLE 5: IR inactivation reduces adhesion of skin cells to the extracellular matrix (ECM)

### Methods

96-well plates were coated with 50µl of PBS containing laminin (20µg/ml; top panel) collagen (10µg/ml; middle panel), or fibronectin (5µg/ml; bottom panel) for 2 hours at 37°C. Plates were washed twice with PBS and further incubated in 1%BSA/PBS for 2 h (hours) at 37°C. control HaCaT cells or HaCaT cells in which the insulin receptor was inactivated by siRNA were detached using Trypsin-EDTA, counted and seeded. After 60 or 120 min, nonattached cells were removed by aspiration, followed by 10 min incubation in ice-cold PBS. Cells underwent fixation with 200µl of 70% ethanol for 30 minutes. Quantification of retained cells was determined with crystal violet staining and OD₅₅₀ measurement in a microplate reader. Vertical axis: OD₅₅₀, expressed as a percentage of attachment of control cells at 120 min. Horizontal axis: Time (min).

### Results

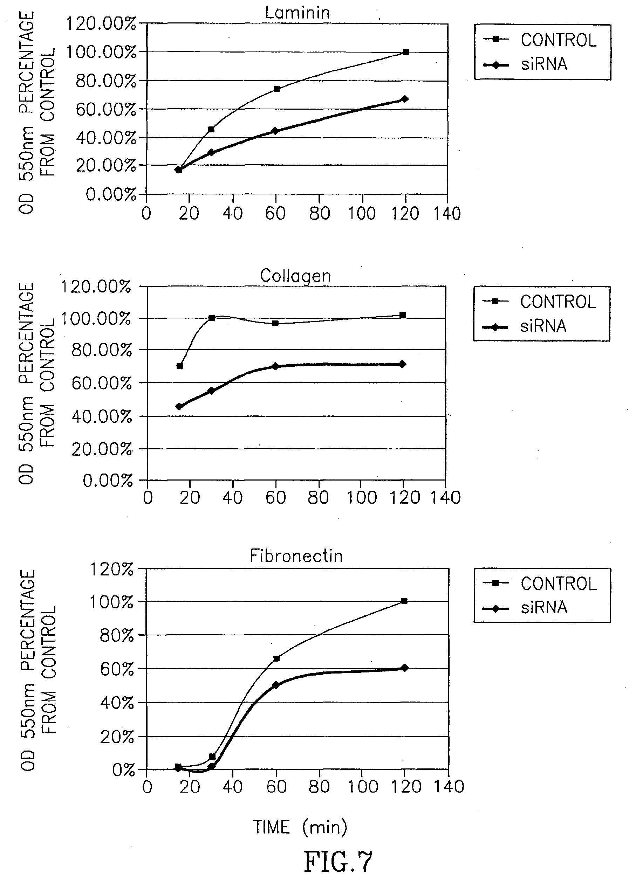

The attachment of the human HaCaT skin cells to the ECM proteins laminin (top panel), collagen (middle panel) and fibronectin (bottom panel) was measured in untreated cells and cells in which IR was inactivated by siRNA. The IR-inactivated cells exhibited decreased attachment relative to the control WT cells (Figure 7).

### EXAMPLE 6: Insulin receptor inactivation inhibits skin transformation in vitro

### Methods

### Ras transformation of skin cells

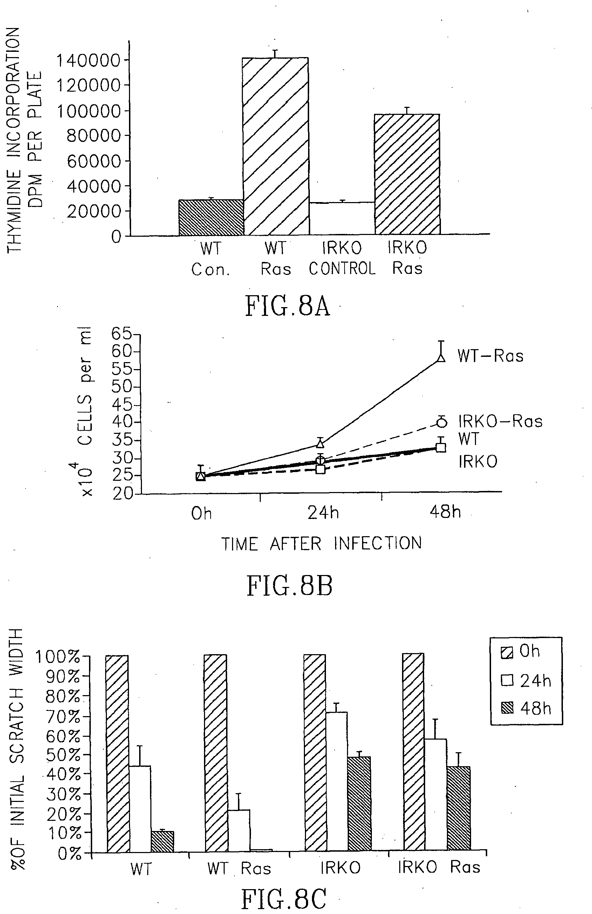

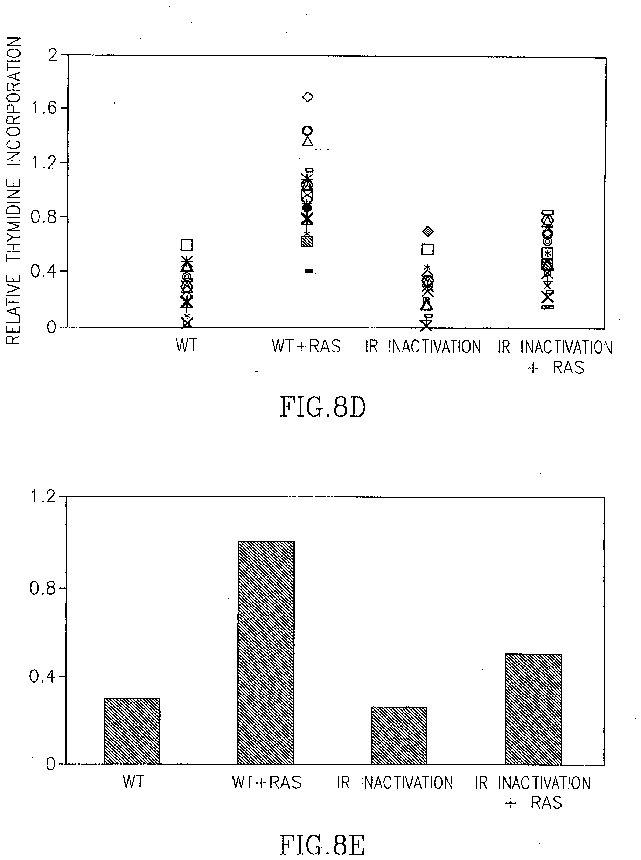

Ras mutations play an important role in skin cancer initiation (Yuspa 1998). Cells were transformed by overexpression of the v-Ha-Ras oncogene. 48hr after v-Ha-Ras retroviral infection, proliferation was determined by [³H]-thymidine incorporation into cellular DNA (Figure 8A). Cellular growth was evaluated as well by detaching the cells from the dish and counting them 24hr and 48hr after retroviral infection (figure 8B).

### Quantifying the number of multi nucleated cells

24 hours post transformation, digital photos were taken, out of which 20 randomly chosen photos were taken from each treatment group and further analyzed to determine the number of multi-nucleated cells in each field. The average of all experiments combined is shown.

### Staining for mitosis anaphase

Cultured primary mouse keratinocytes derived from control or IRKO mice were infected with an *Ha-Ras* expressing vector, and mitosis anaphase was visualized by staining for α/β-tubulin (Cy3) and DAPI (nuclear staining) and fluorescent microscopy.

### Results

IR inactivation resulted in decreased proliferation rate and cellular growth of v-Ha-Ras-transformed IR null skin cells (Figure 8A-B). Furthermore, the cellular migration rate of v-Ha-Ras-transformed IR null keratinocytes was significantly lower compared to transformed cells expressing IR (Figure 8C). The same effect was seen when the IR was inactivated (Figures 8D-E). Inactivation of IR inhibits proliferation of ras transformed skin cells, but has no effect by itself in normal non transformed cells. Individual scores and averages are shown in 8D and 8E, respectively.

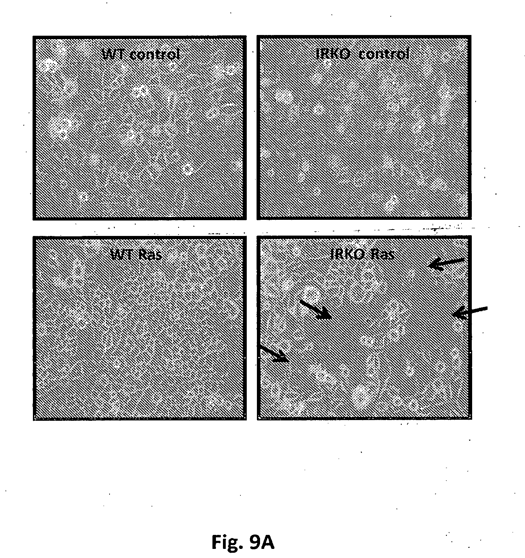

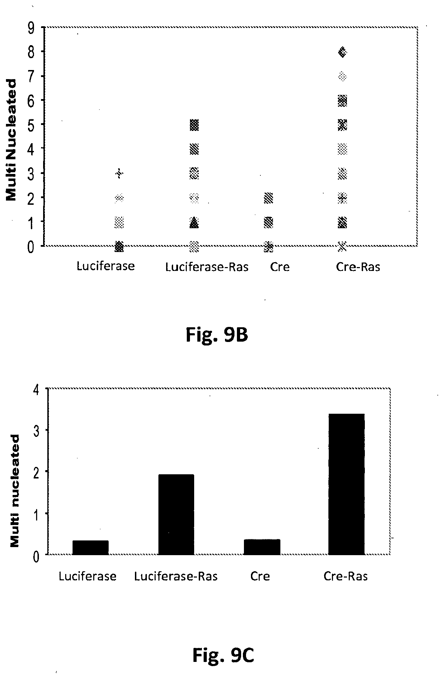

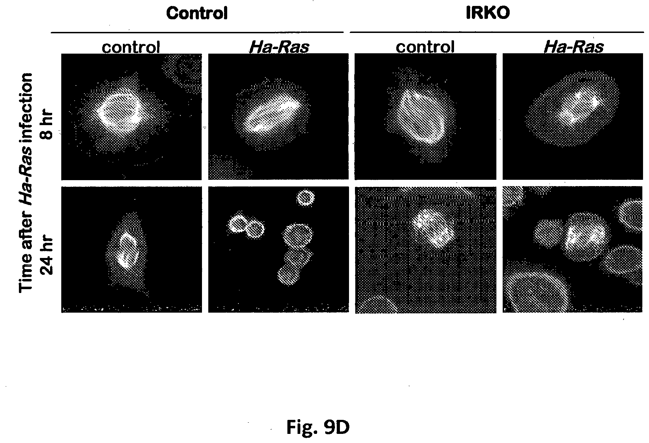

The changes in proliferation were associated with the formation of multi-nucleated cells (Figure 9A). Additionally, primary cells that had been infected *in vitro* with a Cre adeno construct, thus incising out the IR, leading to IR inactivation, were then transformed by infection with ras oncogene. These cells exhibited the appearance of multinucleated cells (Figures 9B and C), providing further evidence that IR inactivation prevents cellular transformation. The above effects resulted from defective mitosis, as evidence by abnormal spindle formation (Figure 9D).

### EXAMPLE 7: Cellular senescence is impaired in IR-null skin cells

### Methods

Primary mouse keratinocytes derived from control and IR-KO mice were infected with Ha-Ras, and 24hr later were treated with TPA [100nM] or vehicle for 24hr. Expression of p21(WAF1/Cip1) was evaluated by Western blot analysis, using a polyclonal antibody. Localization of p21 in the cells was examined by staining for DAPI and p21(WAF1/Cip1) and visualization using fluorescent microscopy.

### Results

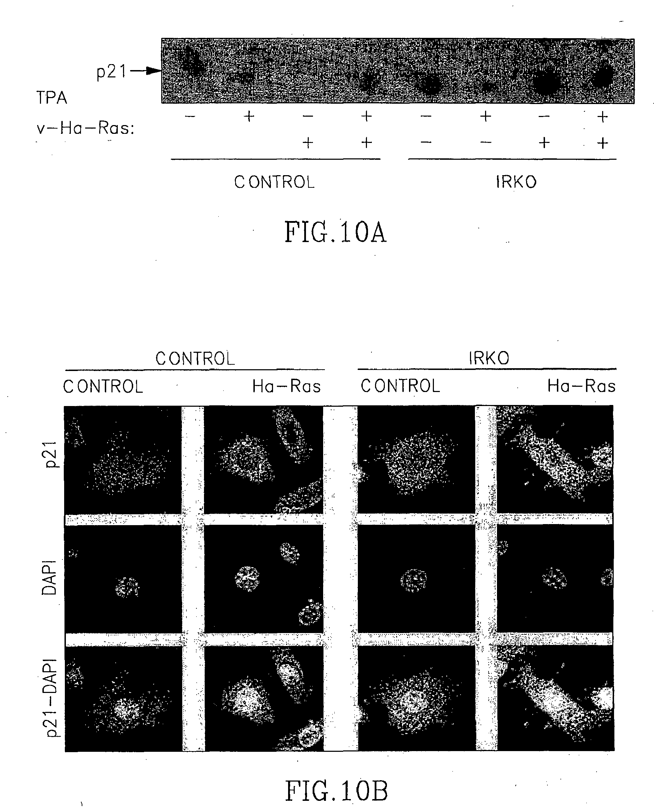

Transformation of control cells using the tumor promoter TPA resulted in decreased levels of p21, a marker of cellular senescence, as expected. However, the expression of p21 remained elevated in transformed IR-null keratinocytes (Figure 10A). Furthermore, while p21 translocated out of the nucleus upon transformation in control cells, p21 remained expressed in both the nucleus and the cytoplasm in the IR-null keratinocytes, in spite of the transformation of the cells (Figure 10B).

### EXAMPLE 8: IR inactivation reverses cellular transformation in vitro

### Methods

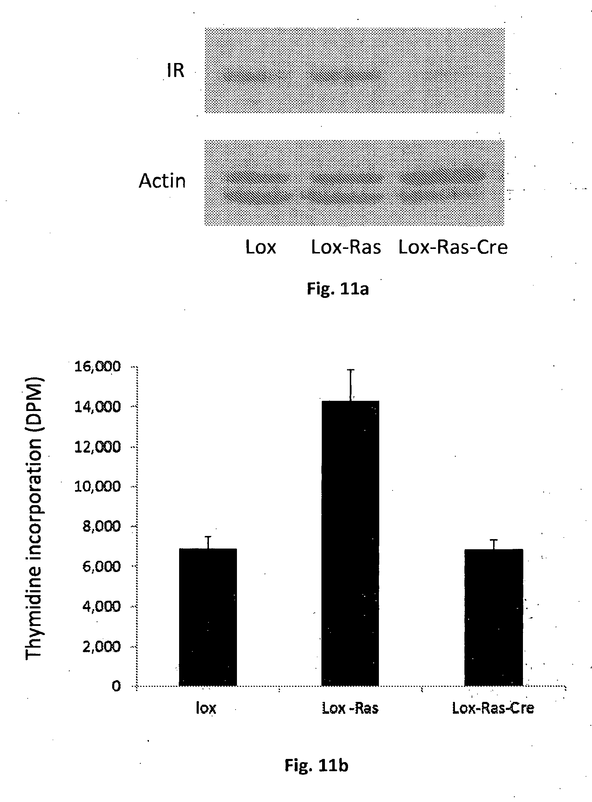

Keratinocytes isolated from the IR-Lox^{+/+} mice were plated in culture dishes and transformed with the Ha-Ras oncogene or not transformed. 24 hours post-transformation, the IR was inactivated by infecting the cells with an adenoviral construct of Cre recombinase (Lox-Ras-Cre). IR inactivation was confirmed by Western blotting **(****Figure 11A****)**, and 24 hours post-IR inactivation, cell division rate was evaluated **(****Figure 11B****)**. Cellular proliferation rate of control, Ras-transformed- and Ras-transformed IR null cells, as determined by [³H]-thymidine incorporation into cellular DNA.

### Results

IR inactivation, demonstrated in Figure 11A, reduced the proliferation rate of the transformed cells (Figure 11B). Thus, IR inactivation reverses cellular transformation and the cells begin to divide like non-cancerous cells.

### EXAMPLE 9: IR expression is required for skin transformation in vivo

### Methods

### Determination of IR expression

IR expression was determined in skin specific insulin receptor knockout (SIRKO) skin cells by Western blotting using specific antibodies against the IR. The blots were re-blotted with an anti-actin antibody, serving as a loading control.

### Immunohistochemistry

Skin was fixed in 4% paraformaldehyde, paraffin embedded, and sectioned (5µm). Sections were stained with H&E. Bright field photographs of representative fields were taken with an Axiovert 200 Zeiss light microscope.

### Induction of tumorigenesis

Tumorigenesis was induced using the two-step DMBA/TPA chemical carcinogenesis protocol (Yuspa 1986, Zoumpourlis 2003) on the skin of the SIRKO mice. A single dose of the carcinogenesis initiator agent DMBA (7,12-dimethylbenz[*a*]anthracene; 100 microgram) was administered, followed by weekly applications of phorbol ester TPA (12-O-tetradecanoylphorbol-13-acetate; 50 microgram).

### Determination of the proliferative index in the tumors

Positively stained cells in PCNA-stained papillomas obtained from control and SIRKO mice sections were counted, and the ratio of proliferating cells to total cells was calculated.

### Results

The SIRKO mouse was used to further study the role of insulin *in vivo*. In this model, using the Cre-Lox system (Mauvais-Jarvis 2002; Sauer 1998), the IR is inactivated only in the epidermal compartment of the skin, while in all other body tissues, including the dermis, the IR is expressed normally. In Figure 12A IR expression in SIRKO proliferating skin cells was detected by Western blotting. A representative experiment of 3 separate replications is shown. A size marker of 100kD is shown. Figure 12B shows H&E staining of skin of 2-day-old control (IRlox/lox) and SIRKO mice. A representative section is shown. Figure 12C shows IR inactivation as demonstrated *in vivo* by immunohistochemistry in skin sections from 2-day-old control (IRlox/lox) and SIRKO mice. Figure 12D shows Western blot analysis of protein extracts prepared from primary cultured keratinocytes and fibroblasts of 2-month-old control and SIRKO mice (two animals per genotype) using an IR-specific antiserum. Both samples are shown for each cell type. Figure 12E shows intact epidermal layer was separated from the dermal layer of control and SIRKO mice by trypsinization, and the intact epidermises were analyzed as described in Figure 12D for the expression of the IR. Figure 12F shows protein extracts of various tissues isolated from control and SIRKO mice were prepared and analyzed as described in Figure 12D.

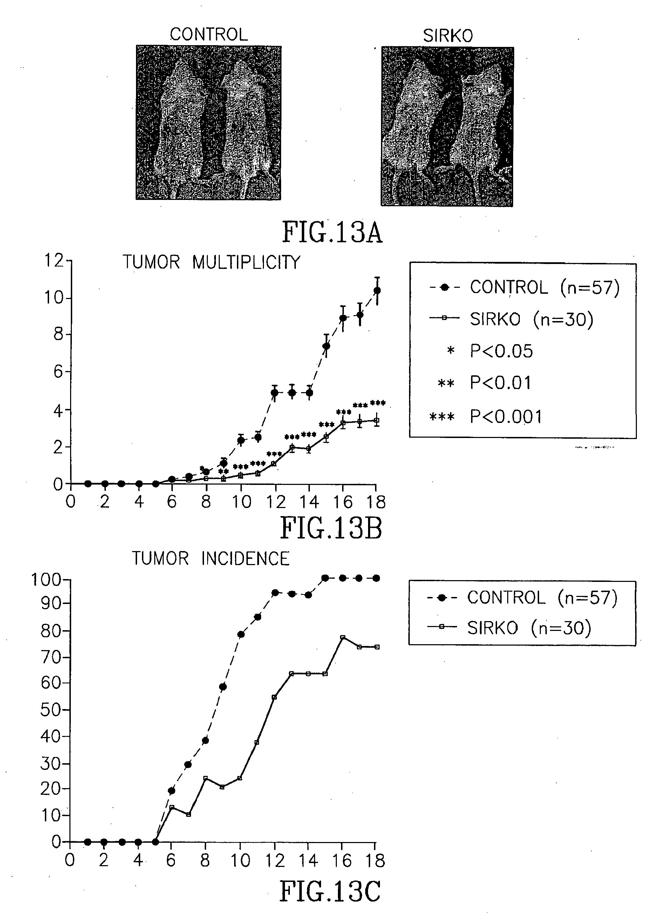

*In vivo*, the SIRKO mice developed significantly fewer tumors compared to control mice, starting at week 8. Figure 13A shows photos of 8-week-old control (lox+/+ cre-/-; left panel) and SIRKO (lox+/+ cre +/-; right panel) mice were shaved on the dorsal region, and a single dose of DMBA was topically applied, followed by twice-weekly application of TPA over 18 weeks. Tumor formation was quantified weekly as shown in Figure 13B and Figure 13**C**. Error bars represent standard error. Tumor multiplicity (Figure 13B) and Tumor incidence (Figure 13C) were markedly decreased; over 95% of the mice developed tumors in the control group whereas over 50% in the SIRKO group did not develop tumors at all.

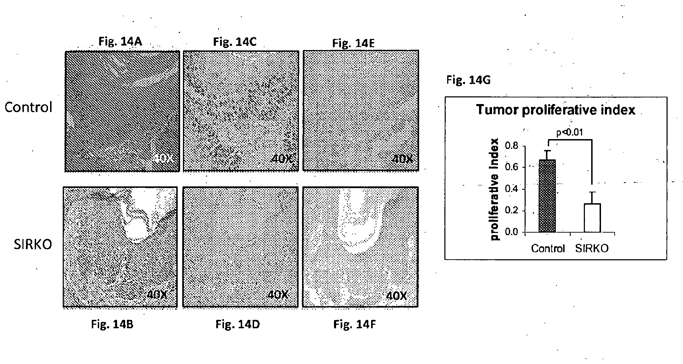

Furthermore, tumors of SIRKO mice exhibited decreased dysplasia and lower proliferative capacity compared to tumors of control mice (Figure 14). Representative sections of papillomas from control (Figures 14A,14C, 14E) and SIRKO (Figures 14B, 14D, 14F) mice at week 18 are shown. H&E staining (Figures 14A/B) revealing smaller size of SIRKO papillomas (**B**). PCNA-stained papillomas (Figures 14**C**/D); dark nuclei indicating less proliferating cells in SIRKO. Keratin-1 immunohistochemistry (Figures 14E/F); dark staining demarcates stronger differentiation of SIRKO papillomas. (Figure 14G) Proliferative index of tumors developed by SIRKO mice and control group demonstrates a marked decrease in the proliferative index in the tumors developed on the SIRKO skin.

### EXAMPLE 10: IR inhibition by a blocking antibody impairs proliferation of human squamous cell carcinoma (SCC) cancer cells

### Methods

### Blocking the IR by a blocking anti IR antibody

SCC Cal 27 cells were grown in culture to 70% confluency. The cells were serum starved for 24 hours after which a blocking anti IR antibody (Thermo Scientific; Insulin Receptor (α-Subunit) Ab-3 (Clone 47-9); Cat. #MS-633-PABX) was added at the concentrations indicated, with or without insulin as indicated, and the cells were incubated further for 24 hours.

### Results

An anti-insulin antibody directed against the alpha subunit was added to squamous cell carcinoma (SCC) cancer cells and the proliferation of the cells was evaluated by thymidine incorporation Figure 15A shows a representative photograph of cells treated with 20nM anti insulin antibody for 24 hours compared to control cells. Figure 15B shows inhibition of IR by an anti-insulin antibody caused inhibition of SCC cancer cell proliferation. Left, center, and right bars are insulin, 10nM, and 20nM respectively. Figure 15C shows that while insulin increased the proliferation of SCC cancer cells, the anti IR blocking antibody reduced the insulin induced proliferation of the human skin cancer cells. In each case, results were normalized to control groups. The P values for 10nM and 20nM are as follows: control vs 10 nM Ab: p = 8.616 E-5; control vs 20 nM Ab: p = 1.745 E-5; control vs 10 nM Ab + insulin: p = 0.00072; control vs 20 nM Ab + insulin: p = 0.00558.

### EXAMPLE 11: The turnover of the SIRKO skin is pathologically accelerated

### Methods

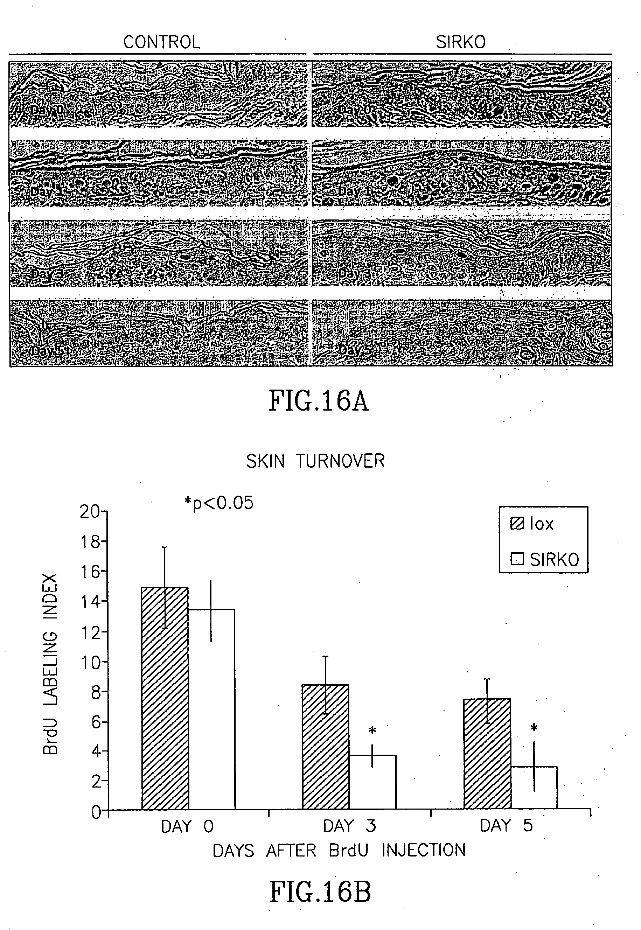

8-week-old SIRKO and control mice were injected intraperitoneally with BrdU, and BrdU positive cells were detected in skin section of the back skin epidermis at several time points as indicated in Figure 16. Exit time of the keratinocytes from basal layer of the epidermis was determined by calculating the percentage of BrdU-positive nuclei in the basal layer of the epidermis compared to the total number of interfollicular basal cells (BrdU labeling index).

### Results

To determine the effect of IR inactivation the turnover rate of skin, the turnover rate of the SIRKO epidermal keratinocytes was measured by BrdU labeling, with an initial 2-hour pulse of Brd-U to label proliferating keratinocytes in the basal layer of epidermis. The fate of these cells was followed over the next 5 days. Figure 16A shows morphology of BrdU-positive cells (dark-stained nuclei) in the back skin epidermis of control and IRKO mice were injected intraperitoneally with BrdU, after 2 h (day 0), and at 1, 3, and 5 days. Figure 16B shows percentage of BrdU-positive nuclei in the basal layer of the epidermis compared to the total number of interfollicular basal cells (BrdU labeling index; vertical axis), after 2 h, and at 3 and 5 days (horizontal axis).

As can be seen there, the exit of keratinocytes from the basal layer was accelerated in the SIRKO epidermis (Figure 16A). Starting on day 3 after BrdU injection, the percentage of BrdU-positive cells was reduced in the SIRKO epidermis compared with the control by over 50% (Figure 16B, p<0.05), indicating an accelerated turnover rate of SIRKO mice epidermis.

### EXAMPLE 12: IR inactivation leads to fragmented and abnormal keratin structure

### Methods

### Electron microscopy analysis

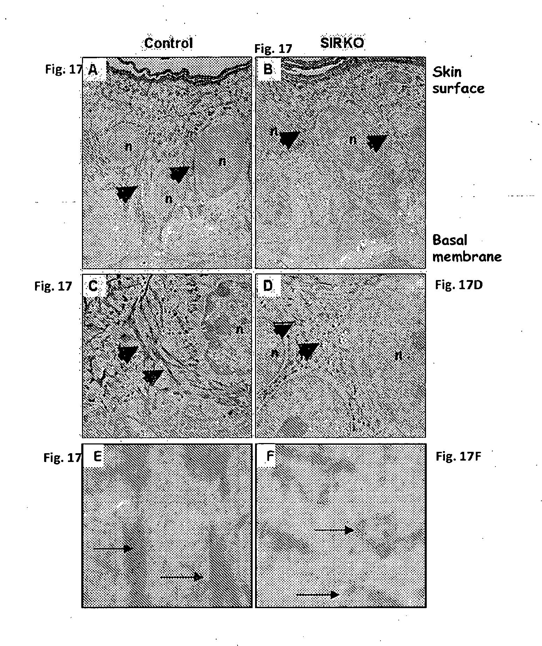

Ear skin sections were isolated from IR(lox/lox) control (Figures 17A,C) and SIRKO (Figures 17B,D) mice, fixed and processed for transmission EM, following standard procedures.

### Immunogold EM analysis

Skin sections were isolated from IR(Iox/Iox) control and SIRKO mice. Sections were fixed and processed for transmission EM, following standard procedures. Immunogold staining was carried out using an anti-K1 antibody. Morphometric analysis of the staining pattern determined that the density of gold particles per protein is similar in the SIRKO and control IR(Iox/Iox) mice and that the staining was specific in both SIRKO and control sections (less than 1.4% of the gold particles were detected in non-protein areas).

### Immunofluorescence analysis

Cells were grown on coverslips and visualized by indirect immunofluorescence using a rabbit anti-mouse K1 antibody and Cy3-labeled goat anti-rabbit IgG as the primary and secondary antibodies. Fluorescence signals were analyzed by (×63).

### Results

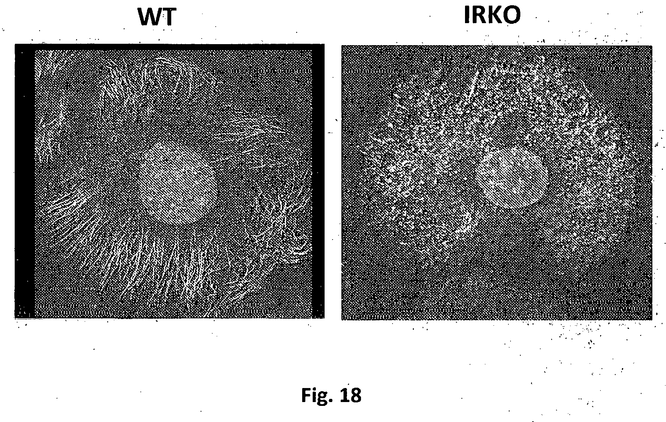

In order to elucidate the changes occurring in skin due to IR inactivation, the ultrastructure of the cells was followed by electron microscopy (EM). The EM studies revealed a striking abnormality in the cytoskeletal intermediate filaments (IF), which appeared abnormally short and fragmented in the SIRKO skin relative to their structure in control mice (Figures 17A-D). Similar results were obtained in IR-null primary cultured skin cells. The skin surface (surface) and basal membrane (BM) are indicated for orientation. IF are marked by arrows, and cell nuclei are marked as well (n). Magnification x 2,700 (A,B) or x5,000 (Figures 17C,D). Photos show representative sections of sections processed from 10 control mice and 10 SIRKO mice. Immunogold studies with anti-K1 antibody were performed to test whether the abnormal filaments contained keratin-1 (K1). These studies showed that K1/K10 proteins were present (Figures 17E-F). The abnormal structure of K1-containing filaments was also confirmed by immunofluorescence studies of IR null skin cells using anti-K1 antibodies (Figure 18). Shown is a representation of confocal microscopy analysis showing K1 protein in primary WT (Figure 18, left panel) and SIRKO (Figure 18, right panel) murine keratinocytes. As can be seen, the K1 bundles were short in the IR-null cells and did not form a net of filaments as seen in the WT cells (Figure 18).

### EXAMPLE 13: Insulin regulation of K1 assembly in murine and human skin cells

### Methods

### Analysis of keratin assembly

Primary skin cells were isolated from the SIRKO and IR(lox/lox) control mice and grown in culture. On day 3, the calcium concentration in the growth medium was raised from 0.05mM to 0.12 mM to induce differentiation, and the culture was further maintained. On day 6, cells were harvested. To evaluate the effects of IR inactivation on the state of keratin filaments, cell lysates were fractionated into a Triton X-100-soluble fraction, containing the elementary keratin units, and a Triton-insoluble fraction, containing the mature keratin filaments. Western blot analysis was carried out on these fractions to estimate the amount of K1 present in each fraction, using Actin content as a loading control. Keratin 14 expression, a marker of the proliferative state, was studied in order to demonstrate specificity the effect. A representative experiment of 4 repeats is depicted in the figures.

### Results

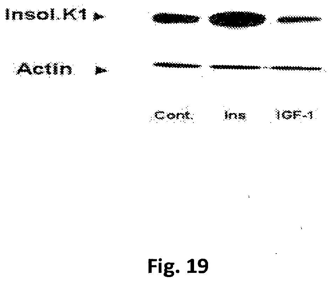

In order to demonstrate that insulin regulates keratin 1 assembly, skin cells were induced to differentiate in the presence of either insulin or IGF1, after which the expression of keratin 1 in the insoluble fraction was evaluated. As can be seen, insulin treatment led to enhanced assembly of K1 (Figure 19), whereas IGF1 led to the opposite effect demonstrating that the effect of insulin on skin cytoskeleton is unique and in contrast to the effects of IGF1.

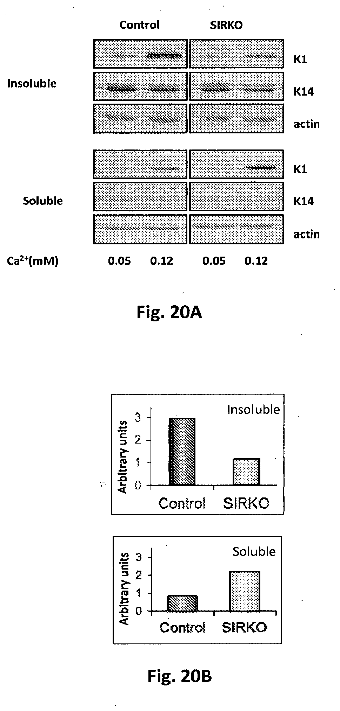

For demonstrating the role of insulin receptor in the assembly process of keratin 1, The IR-null SIRKO skin cells exhibited an increase in soluble K1 compared to the cytoskeletal fraction (Figure 20). The results of a representative western blot analysis are shown (Figure 20A). The results of Western blot analysis of the 4 repeats of each of the fractions obtained were quantified by densitometry, and the expression of K1 was normalized to actin level of each experiment individually. The combined results are presented (Figure 20B). This change in K1 expression and assembly in the IR null cells confirms that K1 filament assembly is abnormal in SIRKO cells and that the IR is involved directly in the regulation of K1.

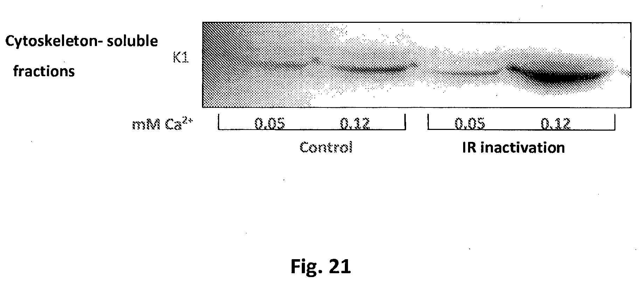

In another model system, IR was inactivated *in vitro* after isolating primary cells from IR floxed mice. IR-inactivated keratinocytes from IR-floxed mice overexpressed a Cre recombinase construct, while control cells were infected by a luciferase construct. Cells were induced to differentiate by increasing the calcium concentration in the growth medium to 0.12mM, and after 24 hours the expression of keratin-1 was determined in the cellular soluble fraction. A Western blot of the cytoskeleton-soluble fraction, showing keratin-1 expression, is depicted. In this model as well, IR inactivation increased the proportion of K1 in the soluble fraction (Figure 21), further confirming the role of IR in the regulation of K1 assembly.

### EXAMPLE 14: Treatment with IR-specific siRNA Disrupts K1 Assembly

### Methods

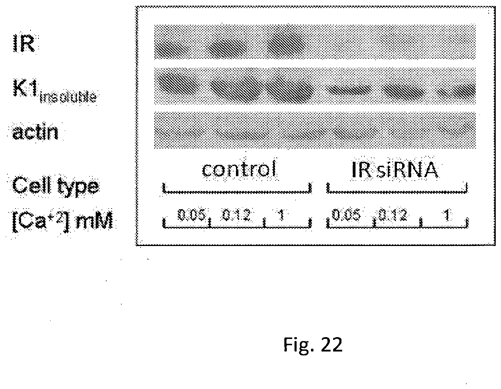

IR expression in HaCaT cells was inactivated by IR siRNA. Differentiation of HaCaT WT or IR-inactivated cells by siRNA was induced by changing the Ca²⁺ levels in the medium (0.05mM\0.12mM\1mM) for 24 hours. Keratin-1 expression of K1 (Figure 22, middle panel) was determined in the insoluble fraction, as described above, using actin expression (Figure 22, lower panel) as a loading control. A representative experiment from 4 independent experiments is shown.

### Results

To test whether abnormal K1 assembly could be induced by knocking down IR expression, an IR-specific siRNA was overexpressed in HaCaT cells (human epidermal keratinocytes). A significant disruption was seen in K1 assembly (Figure 22).

### EXAMPLE 15: Use of antibodies that inhibit IR signaling for skin cancer therapy and prevention

Antagonistic anti-IR antibodies are tested for ability to reverse transformation of tumors in vivo and regulate their proliferation and/or differentiation characteristics, for example as described hereinabove. Tumors are induced in experimental animals using the two-step DMBA/TPA chemical carcinogenesis protocol (Yuspa et al 1986). The agent is applied to the tumors at various time points before and during tumor initiation or promotion, and tumor progression is monitored. The effects of the agents on human cancers are tested by applying the agents to skin cancer tumors. The agents are applied topically via a cream, an ointment, or a cream-based ointment such as J&J baby lotion (J&J, New Brunswick, NJ). In alternate experiments, the agents are injected epicutaneously, subcutaneously, intradermally, or intratumorally. In another experiment, the agent is administered orally.

Tumor incidence, mass (e.g. by measuring tumor diameter) prevalence is followed, after which mice are sacrificed, tumors are weighed and tumor biopsies are analyzed by histopathology. Defined characteristics of tumor progression are measured, for example histopathological tumor appearance; cell proliferation by PCNA or BrdU (injected prior to sacrifice); suprabasal proliferation of cells disconnected from the basement membrane, a process that is characteristic of progression; altered epidermal differentiation, including reduction in expression of normal epidermal keratin 1 and keratin 10; aberrant expression of keratin 13; and induction of expression of keratin 8/18, K7 and/or K19, which are characteristic of skin and other epithelial malignancies.

One of skill will appreciate that IR receptor inhibitory peptides can be used in place of antagonistic anti-IR antibodies as used in this example.

### EXAMPLE 16: Use of antibodies that inhibit IR signaling for skin cancer therapy and prevention in a human xenograft model

Antagonistic anti-IR antibodies are tested for ability to reverse transformation of keratinocytes by measuring their proliferation and/or differentiation, for example as described herein. Methods similar to Morton CL et al (Nat Protocols. 2007;2(2):247-50) can be utilized. Establishment of human tumor xenografts in immunodeficient mice), cell lines of human SCC or BCC cancer are grown in complete medium until they reach 70-80% confluence. Prior to harvesting, the medium may be replaced with fresh medium to remove dead and detached cells. The cells are washed (e.g. with PBS) and re-suspended. Non viable cells may be excluded by e.g. trypan blue staining.

Cells are mixed and a set inoculum (e.g. 3-10 x 10⁶ cells) is injected on to athymic nude/ scid mice (4-8 months old) subcutaneously (s.c.) into the lower flank of the mice.

At time of injection and at several time points following injection, the agent is administered to the mice xenografts topically, subcutaneously, intradermally or (once a visible mass appears) intratumorally.

Tumor incidence, mass (e.g. by measuring tumor diameter) are prevalence is followed, after which mice are sacrificed, tumors are weighed and tumor biopsies are analyzed by histopathology. Defined characteristics of tumor progression are measured, for example histopathological tumor appearance; cell proliferation by PCNA or BrdU (injected prior to sacrifice); suprabasal proliferation of cells disconnected from the basement membrane, a process that is characteristic of progression; altered epidermal differentiation, including reduction in expression of normal epidermal keratin 1 and keratin 10; aberrant expression of keratin 13; and induction of expression of keratin 8/18, K7 and/or K19, which are characteristic of skin and other epithelial malignancies.

One of skill will appreciate that IR receptor inhibitory peptides can be used in place of antagonistic anti-IR antibodies as used in this example.

### EXAMPLE 17: Use of anti-IR siRNA agents for skin cancer therapy and prevention

siRNA agents against IR are tested for ability to reverse transformation of keratinocytes by measuring their proliferation and/or differentiation, for example as described hereinabove. Tumors are induced in experimental animals using the two-step DMBA/TPA chemical carcinogenesis protocol (Yuspa et al 1986). The agent is applied to the tumors at various time points before and during tumor initiation or promotion, and tumor progression is monitored. The effects of the agents on human cancers are tested by applying the agents to skin cancer tumors. The agents are applied topically via a cream, an ointment, or a cream-based ointment such as J&J baby lotion (J&J, New Brunswick, NJ). In alternate experiments, the agents are injected epicutaneously, subcutaneously, intradermally, or intratumorally. In another experiment, the agent is administered orally.

Tumor incidence, mass (e.g. by measuring tumor diameter) prevalence is followed, after which mice are sacrificed, tumors are weighed and tumor biopsies are analyzed by histopathology. Defined characteristics of tumor progression are measured, for example histopathological tumor appearance; cell proliferation by PCNA or BrdU (injected prior to sacrifice); suprabasal proliferation of cells disconnected from the basement membrane, a process that is characteristic of progression; altered epidermal differentiation, including reduction in expression of normal epidermal keratin 1 and keratin 10; aberrant expression of keratin 13; and induction of expression of keratin 8/18, K7 and/or K19, which are characteristic of skin and other epithelial malignancies.

### EXAMPLE 18: Use of IR siRNA agents for skin cancer therapy and prevention in a human xenograft model

siRNA agents against IR are tested for ability to reverse transformation of keratinocytes by measuring their proliferation and/or differentiation, for example as described hereinabove.

Methods similar to Morton CL et al (Nat Protocols. 2007;2(2):247-50) can be utilized. Establishment of human tumor xenografts in immunodeficient mice), cell lines of human skin cancer (e.g. SCC and BCC) are grown in complete medium until they reach 70-80% confluence. Prior to harvesting, the medium may be replaced with fresh medium to remove dead and detached cells. The cells are washed (e.g. with PBS) and re-suspended. Non viable cells may be excluded by e.g. trypan blue staining.

Cells are mixed and a set inoculum (e.g. 3-10 x 10⁶ cells) is injected on to athymic nude/ scid mice (4-8 months old) subcutaneously (s.c.) into the lower flank of the mice.

At time of injection and at several time points following injection, the agent is administered to the mice xenografts topically, subcutaneously, intradermally or (once a visible mass appears) intratumorally.

Tumor incidence, mass (e.g. by measuring tumor diameter) are prevalence is followed, after which mice are sacrificed, tumors are weighed and tumor biopsies are analyzed by histopathology. Defined characteristics of tumor progression are measured, for example histopathological tumor appearance; cell proliferation by PCNA or BrdU (injected prior to sacrifice); suprabasal proliferation of cells disconnected from the basement membrane, a process that is characteristic of progression; altered epidermal differentiation, including reduction in expression of normal epidermal keratin 1 and keratin 10; aberrant expression of keratin 13; and induction of expression of keratin 8/18, K7 and/or K19, which are characteristic of skin and other epithelial malignancies.

### References

Boukamp P., Petrussevka RT., Breitkreutz D., Hornung J., Markham A., Fusenig NE. (1988) Normal keratinization in a spontaneously immortalized aneuploid human keratinocyte cell line. J. Cell Biol. 106:761-771
Breitkreutz, D., Bohnert, A., Herzmann, E., Bowden, P. E., Boukamp, P. and Fusenig, N. E. (1984). Differentiation specific functions in cultured and transplanted mouse keratinocytes: environmental influences on ultrastructure and keratin expression. Differentiation. 26, 154-169. Breitkreutz D, Stark HJ, Plein P, Baur M, Fusenig NE: Differential modulation of epidermal keratinization in immortalized (HaCaT) and tumorigenic human skin keratinocytes (HaCaT-ras) by retinoic acid and extracellular Ca2+. Differentiation. 54:201-217, 1993.
Fuchs E: Epidermal differentiation: the bare essentials. J. Cell Biol. 111:2807-2814, 1990.
Jansen B, Zangemeister-Wittke U. Antisense therapy for cancer-the time of truth. Lancet Oncol. 2002 Nov;3(11):672-83.
Mauvais-Jarvis, F, Kulkarni, RN, Kahn, CR: Knockout models are useful tools to dissect the pathophysiology and genetics of insulin resistance. Clin.Endocrinol.(Oxf) 57:1-9, 2002.
Sadagurski, M, Nofech-Mozes, S, Weingarten, G, White, MF, Kadowaki, T, Wertheimer, E: Insulin receptor substrate 1 (IRS-1) plays a unique role in normal epidermal physiology. J. Cell Physiol. 213:519-527, 2007.
Sauer, B: Inducible gene targeting in mice using the Cre/lox system. Methods 14:381-392, 1998.
Schäffer L, Brissette RE, Spetzler JC, Pillutla RC, Østergaard S, Lennick M, Brandt J, Fletcher PW, Danielsen, GM, Hsiao KC, Andersen AS, Dedova O, Ribel U, Hoeg-Jensen T, Hansen PH, Blume AJ, Markussen J, Goldstein NI. Proc Natl Acad Sci U S A. 2003; 100(8):4435-9. Assembly of high-affinity insulin receptor agonists and antagonists from peptide building blocks.
Wang H, Prasad G, Buolamwini JK, Zhang R. Antisense anticancer oligonucleotide therapeutics. Curr Cancer Drug Targets. 2001 Nov;1(3):177-96.
Yuspa, SH: Cutaneous chemical carcinogenesis. J.Am.Acad.Dermatol. 15:1031-1044, 1986
Yuspa SH, Kilkenny AE, Steinert PM, Roop DR: Expression of murine epidermal differentiation markers is tightly regulated by restricted extracellular calcium concentrations in vitro. J. Cell Biol. 109:1207-1217, 1989.
Yuspa SH: The pathogenesis of squamous cell cancer: lessons learned from studies of skin carcinogenesis. J. Dermatol Sci. 17:1-7, 1998.
Zhou P, Ten S, Sinha S, Ramchandani N, Vogiatzi M, Maclaren N. Insulin receptor autoimmunity and insulin resistance. J Pediatr Endocrinol Metab. 2008; 21(4):369-75.
Zoumpourlis, V, Solakidi, S, Papathoma, A, Papaevangeliou, D: Alterations in signal transduction pathways implicated in tumour progression during multistage mouse skin carcinogenesis. Carcinogenesis 24:1159-1165, 2003

## Claims

1. A composition comprising an active agent that distinctly inhibits the insulin receptor and/or distinctly reduces expression of the insulin receptor for use in a method for treating or preventing skin cancer, a pre-cancerous or hyperplastic skin lesion, basal cell carcinoma, squamous cell carcinoma, or actinic keratosis,
wherein the active agent comprises
- an insulin receptor-specific antibody,
- an insulin receptor antagonist peptide or a peptide capable of blockading the interaction between insulin and IR, and/or
- an RNAi agent that reduces expression of the insulin receptor gene and/or IR protein levels.

2. The composition for the use according to claim 1, wherein the active agent is a humanized insulin receptor-specific antibody.

3. The composition for the use according to claim 1, wherein said composition is a topical composition.

## Patentansprüche

1. Eine Zusammensetzung umfassend einen Wirkstoff, welcher deutlich den Insulinrezeptor hemmt und/oder deutlich die Expression des Insulinrezeptors reduziert, zur Verwendung in einem Verfahren zur Behandlung oder zur Vorsorge von Hautkrebs, einer präkanzerösen oder hyperplastischen Hautveränderung, Basaliom, Plattenepithelkarzinom oder aktinische Keratose,
wobei der Wirkstoff
- einen für den Insulinrezeptor spezifischen Antikörper,
- ein Insulinrezeptor-Antagonist-Peptid, oder ein Peptid, welches in der Lage ist, die Interaktion zwischen Insulin und dem Insulinrezeptor zu blockieren, und/oder
- einen RNAi Wirkstoff, der die Expression des Insulinrezeptorgens und/oder den Proteinspiegel des Insulinrezeptors reduziert,
umfasst.

2. Die Zusammensetzung zur Verwendung gemäß Anspruch 1, wobei der Wirkstoff ein für den Insulinrezeptor spezifischer humanisierter Antikörper ist.

3. Die Zusammensetzung zur Verwendung gemäß Anspruch 1, wobei die Zusammensetzung eine topische Zusammensetzung ist.

## Revendications

1. Composition comprenant un agent actif qui inhibe distinctement le récepteur d'insuline et/ou réduit distinctement l'expression du récepteur d'insuline pour une utilisation dans une méthode de traitement ou de prévention du cancer de la peau, une lésion cutanée précancéreuse ou hyperplasique, un carcinome basocellulaire, un carcinome épidermoïde, un carcinome épidermoïde, ou une kératose actinique,
dans laquelle l'agent actif comprend
- un anticorps spécifique du récepteur d'insuline,
- un peptide antagoniste des récepteurs d'insuline ou un peptide capable de bloquer l'interaction entre l'insuline et le récepteur d'insuline (IR), et/ou
- un agent ARNi qui réduit l'expression du gène du récepteur d'insuline et/ou les taux de protéines d'IR.

2. Composition pour l'utilisation selon la revendication 1, dans laquelle l'agent actif est un anticorps spécifique du récepteur d'insuline humanisé.

3. Composition pour l'utilisation selon la revendication 1, dans laquelle ladite composition est une composition topique.
